# EUROPEAN PATENT APPLICATION

(11) **EP 4 279 480 A1**
(43) Date of publication of application: **22.11.2023**
(21) Application number: 22382472.3
(22) Date of filing: 17.05.2022
(51) Int. Cl.: C07C 69/743, C07C 211/63, C07C 229/46, C07C 271/28, C07D 213/64, C07D 223/28, C07D 405/12, C07D 495/04, C07J 1/00, C07D 239/10, C07C 69/747, C07C 69/75, C07C 69/757

(54) **CYCLOPROPENIUM COMPOUNDS, PROCESS FOR THEIR PREPARATION AND USE**

(71) Applicant: Fundació Institut Català d'Investigació Química, 43007 Tarragona (ES)
(72) Inventor: GARCÍA SUERO, Marcos, E-43005 Tarragona (ES); TU, Hangfei, 43002 Tarragona (ES); JEANDIN, Aliénor, 43001 Tarragona (ES)
(74) Representative: Pons

(57) **Abstract**

The present invention relates to a cyclopropenium salt bearing an electron withdrawing group suitable for use as a reagent in cyclopropenylation reactions of formula (I). The invention also relates to a process for their preparation, the use thereof as cyclopropenylation reagent, to an electrophilic aromatic substitution process of cyclopropenylation, and to a process for cyclopropenylation of organometallic substrates using said reagents.

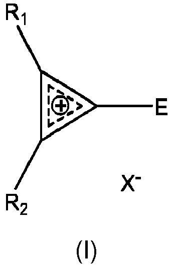

## Description

### FIELD OF THE INVENTION

The present invention relates to a cyclopropenium salt bearing an electron withdrawing group suitable for use as a reagent in cyclopropenylation reactions. The invention also relates to a process for their preparation, the use thereof as cyclopropenylation reagent and to an electrophilic aromatic substitution process of cyclopropenylation.

### BACKGROUND

The cyclopropane ring is a structural motif broadly used in medicinal chemistry. Tanaji T. Talele has reviewed in J. Med. Chem. 2016, 59, 19, 8712-8756 the role of this fragment in this scientific field. Indeed, the cyclopropane ring influences notable pharmaceutical properties such as intrinsic lipophilicity, metabolic stability, alteration of the pKa, and binding to target proteins among others. According to Bauer and co-workers (RSC Med Chem. 2021; 12(4): 448-471), these advantages are attributed to the planar, rigid structure and significant ring strain of the cyclopropyl ring (27.5 kcal mol⁻¹). In the cyclopropyl ring, the internal C-C bonds are often considered pseudo double bonds, while retaining a bond length closer to an aliphatic C-C bond. In addition, the C-H bonds have more s-character and are shorter, stronger and more polarised than conventional C-H bonds. These unique properties make the cyclopropyl ring an attractive substituent for medicinal chemistry applications.

In contrast, the cyclopropenyl ring has a carbon-carbon double bond, whereby the carbon atoms are in sp² hybridization state, thereby increasing significantly the ring strain of the cyclopropene ring if compared with the cyclopropane ring. Nevertheless, the cyclopropenyl structural motif has comparably barely been used in medicinal chemistry. The reasons for this fact are twice. In a first place, cyclopropenyl compounds are difficult to prepare because the cyclopropenyl ring is not thermodynamically favoured. As a consequence of this, cyclopropenyl compounds tend to be reactive and prompt to degradation. Consequently, and despite the cyclopropenyl group being a potentially attractive motif for medicinal chemistry applications among others, there is a need for reagents and methods for the preparation of cyclopropenylated compounds.

Several methods for the synthesis of cyclopropenylated compounds are known in the art and have been reviewed by Rubin, M. et al in Synthesis 2006, No. 8, pp 1221-1245. The most common synthetic strategies for the preparation of cyclopropenylated compounds are: 1,2-elimination from a cyclopropane precursor possessing a good leaving group; cycloisomerization of vinylcarbenes generated in situ from tosyl hydrazones, diazoalkenes, vinyldiazirines, allyl halides, or epoxides; [2+1] cycloaddition of carbenoids generated from diazocompounds or iodonium ylides to alkynes; photochemical or transition-metal-catalyzed extrusion of nitrogen from 3*H*-pyrazoles; cascade 1,3/1,2-elimination from 1,3-dihalopropanes; and functionalization of preformed cyclopropenes. For instance, the [2+1] cycloaddition of carbenoids generated from diazocompounds to alkynes known in the art can be depicted according to the following scheme: 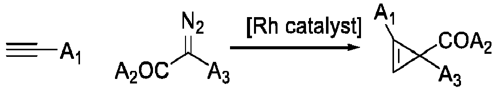 wherein A₂ is OMe, OEt, OtBu, O-Menthyl, or NMe₂. Such methods have been reported by Doyle M.P. in J. Am. Chem. Soc. 1993, 115, 9968, or by Lou, Y. et al in J. Am. Chem. Soc. 2004, 126, 8916. These methods are however limited to terminal alkynes substrates and therefore do not allow the formation of cyclopropene rings whereby the three carbon atoms are substituted. Briones et al. have also shown in Org. Lett. 2011, 13, 3984, that silver triflate is a good catalyst for the preparation of cyclopropenes from internal alkynes using aryldiazo carbenoid compounds substituted with an electron withdrawing group. This cyclopropenylation method requires the use of a metal-containing catalyst and is limited in scope of substrates. Other less common methods known in the art include addition of nucleophiles to cyclopropenium salts.

For instance, Padwa, A. et al disclose in J. Am. Chem. Soc. 1983, 105, 4446 the reaction of diphenylmethylcyclopropenylium perchlorate with 2-vinylbenzene magnesium bromide for the production of 1,2-diphenyl-3-methyl-3-(2-vinylphenyl)cyclopropene. Further examples of additions of nucleophiles to cyclopropenium salts are known in the art and have been reviewed by Komatsu et al. in Chem. Rev. 2003, 103, 1371-1427. In particular, cyclopropenium salts are used in the art in the preparation of cyclopropenylated compounds by reaction with anionic sulfonate salts or organometallic reagents, such as organolithium compounds and Grignard-type reagents. In particular, when the nucleophile is a carbon atom comprised in an aromatic or heteroaromatic ring, such as a carbon atom in a phenyl ring, the procedures known in the art require this carbon atom be activated as a nucleophile by formation of the corresponding organolithium compound or magnesium bromide derivative, suggesting that the cyclopropenylium salt is not electrophilic enough for reacting without previous activation of the nucleophile.

An example of reaction of benzene with a cyclopropenium salt bearing three chlorine atom substituents has been reported in the art by Tobey, S. W. in J. Am. Chem. Soc. 1964, 86, 4215. While the disclosed reaction does not require the preparation of an organometallic derivative of benzene, it uses the leaving group nature of a chlorine atom born by the cyclopropenium salt to enhance its electrophilic character, thus leading to the formation of a gem-dichloro cyclopropenylated compound, according to the following scheme: 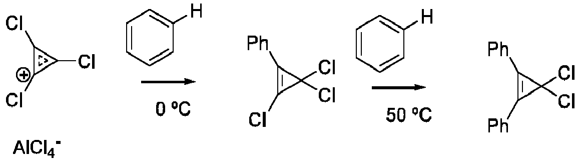 The disclosed reaction thus requires the displacement of a substituent borne by the cyclopropenium salt.

Methods for the preparation of cyclopropenium salts are known in the art and have been extensively reviewed by Komatsu et al. and in Science of Synthesis: Houben-Weyl Methods of Molecular Transformations Vol. 45a:Monocyclic Arenes, Quasiarenes, and Annulenes, Chapter 45.1.1.1, by Merino, P., ISBN 970-1-58890-536-9 (Thieme New York). Said methods include, (i) hydride or halide abstractions from cyclopropene substrates substituted with auxiliary groups such as silyl groups, ferrocene, alkyl; and (ii) Friedel-Crafts reactions involving halogenated cyclopropenium cations. A further procedure was described by Maier, G. in Synthesis 1992, 561 for the preparation of trisilylated cyclopropenium cations from disilylated alkyne compounds and diazo-containing carbenoids which involves three synthetic steps:

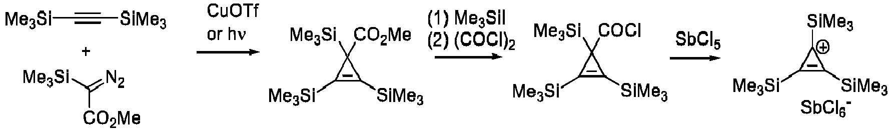

Further carbenoid compounds have been disclosed in the art. For instance, patent application WO 2018/146200 discloses hypervalent iodine compounds bearing a diazomethyl group, whereby the diazomethyl group is substituted with an electron withdrawing group. The disclosed compounds are useful for the reaction of transfer of a diazomethyl group functionalized with an electron withdrawing group to an aromatic or heteroaromatic ring via activation of an aromatic C-H bond. The authors are however silent about the use of these compounds in the preparation of cyclopropenium salts from alkyne derivatives.

Few cyclopropenium salts whereby at least two carbon atoms of the cyclopropenium ring is substituted with a carbon atom are known in the art. Such cyclopropenium salts are for instance those of formula 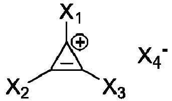 wherein each of X₁, X₂ and X₃ is selected from phenyl, *tert*-butyl, cyclopropyl, 9-tripticyl, and alkynyl substituted with SiMe3 or Si(*i*Pr)3; and X₄ is perchlorate, hexabromotellurate or hexafluoroantimonate. In particular, no cyclopropenium salts whereby one of the carbon atom of the cyclopropenium ring is substituted with an electron withdrawing group, such as an ester group, have been disclosed in the art. On the contrary, cyclopropenium salts bearing electron donating groups have been reported in US 2008 269 525, such as the salt having the cation of formula

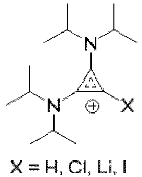

From what is disclosed in the art, it derives that there is still a need for providing improved cyclopropenium compounds useful for the synthesis of cyclopropenylated compounds, in particular of cyclopropenylated aromatic compounds.

### SUMMARY OF THE INVENTION

After exhaustive research, the inventors have developed a novel method for the preparation of cyclopropenium salts bearing an electron withdrawing group, such as an ester group. The method of the invention comprises the step of contacting a non-terminal alkyne compound with a hypervalent iodine reagent bearing a diazomethyl group to which the electron withdrawing group is appended. The cycloaddition of the diazomethyl group, acting as a carbenoid, with the alkyne in the presence of a rhodium(II) catalyst allows forming the corresponding cyclopropenium salt bearing an electron withdrawing group. It is believed that the rhodium(II) catalyst forms a metallacarbene species by nitrogen evolution from the hypervalent iodine reagent. Said metallacarbene intermediate is believed to react by cycloaddition with the alkyne to form a cyclopropenylated intermediate bearing a hypervalent iodine substituent which then eliminates to provide the cyclopropenium salt, according to the following scheme:

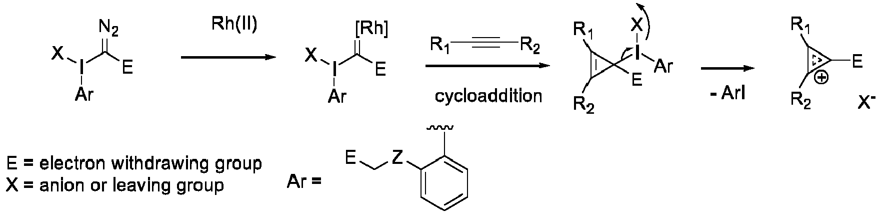

The inventors have found that the presence of an electron withdrawing substituent, such as an ester group, on the cyclopropenium ring activates the resulting salt as electrophile and surprisingly increases its reactivity towards nucleophiles. It was surprisingly found that organic compounds containing aromatic C-H bonds react with the cyclopropenium salt bearing an electron withdrawing group with no need for the activation of the nucleophile by formation of an organometallic reagent, such as an organolithium compound or a Grignard reagent, following the general reaction:

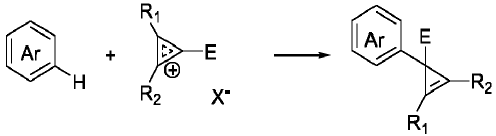

In contrast with other processes disclosed in the art, the preparation of cyclopropenylated aromatic compounds from cyclopropenium salts bearing an electron withdrawing group can advantageously be carried out room temperature and with no need for previously activating the reaction substrate by formation of the corresponding organometallic species. In addition, the electrophilic aromatic substitution takes place with high regioselectivity thanks to the presence of an electron withdrawing group on the cyclopropenium salt which renders the carbon atom adjacent to the electron withdrawing group less electron rich and more susceptible to nucleophilic attack than the other carbon atoms of the cyclopropenium ring. Additionally, no substituent of the cyclopropenium ring is displaced during the aromatic cyclopropenylation process of the invention.

Thus, in a first aspect, the invention relates to a compound of formula (I) 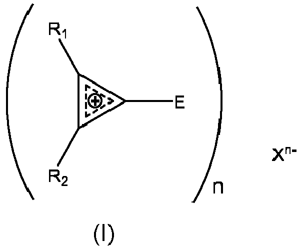
wherein E is an electron withdrawing group selected from the group consisting of (C₁-C₆)haloalkyl, a radical of formula -COOR₃, a formyl group (-CHO), (C₁-C₆)alkylcarbonyl, carboxyl (-COOH), nitro, (C₁-C₆)alkyloxysulfonyl, a group of formula -P(O)(O(C₁-C₆)alkyl)₂, nitrile and an aromatic ring system comprising from 1 to 2 6-membered aromatic rings, the members of the rings being selected from the group consisting of C, CH and N, being at least one member N, and the rings being further optionally substituted at any available position with one or more group selected from halogen, (C₁-C₆)alkyl, (C₁-C₆)haloalkyl, (C₁-C₆)alkyloxy, (C₁-C₆)alkylcarbonyl,(C₁-C₆)alkylcarbonyloxy, (C₁-C₆)alkyloxycarbonyl, nitrile, a formyl group and nitro;
each of R₁, R₂ and R₃ is independently selected from the group consisting of (C₁-C₁₂)alkyl, (C₂-C₁₂)alkenyl and a ring system RS comprising from 1 to 5 rings, each ring being saturated, unsaturated or aromatic, each ring comprising from 3 to 7 members selected from the group consisting of C, CH, CH₂, O, N, NH, NR₄ and S, each ring being isolated, bridged or fused; wherein each of the (C₁-C₁₂)alkyl, (C₂-C₁₂)alkenyl and the ring system RS is optionally substituted at any available position with one or more radicals selected from the group consisting of halogen, (C₆-C₂₀)aryl, (C₅-C₂₀)heteroaryl, (C₃-C₁₂)cycloalkyl, (C₁-C₆)alkyl, (C₁-C₆)haloalkyl, (C₁-C₆)alkyloxy, (C₁-C₆)alkylcarbonyl,(C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a formyl group, a nitro group, a radical of formula -PG₁, a radical of formula -OPG₁ and a radical of formula -NR₄R₅;
wherein PG₁ is selected from the group consisting of *N*-phthalimidyl and a radical of formula -SiR₆R₇R₈;
wherein R₄ is selected from the group consisting of (C₁-C₆)alkyloxycarbonyl, benzyloxycarbonyl, 9-fluorenylmethoxycarbonyl; (C₁-C₆)alkylsulfonyl and phenylsulfonyl wherein the phenyl ring is optionally substituted with one or more radicals selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, nitrile, a formyl group and nitro;
R₅ is hydrogen or (C₁-C₆)alkyl;
each of R₆, R₇ and R₈ is independently selected from the group consisting of (C₁-C₆)alkyl and phenyl;
xⁿ⁻ is an anion selected from the group consisting of hexafluorophosphate, hexafluoroantimonate, hexafluoroaluminate, hexafluorogermanate, hexafluorozirconate, hexafluoroarsenate, hexafluorotitanate, hexachloroantimonate, hexafluorosilicate, tetrafluoroberyllate, hexachlorophosphate, hexachlorosilicate, perchlorate, trifluoromethanesulfonate, tetrafluoroborate and B(Ar)₄⁻ wherein Ar is a phenyl ring optionally substituted at any available position with a group selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, nitrile, a formyl group and nitro;
and n is an integer of from 1 to 3.

The invention also relates to a process for the preparation of the compound of the first aspect of the invention. Thus, in a second aspect, the invention relates to a process for the preparation of a compound of formula (I) as defined in the first aspect of the invention, comprising the step of contacting a compound of formula (II) with a compound of formula (III) 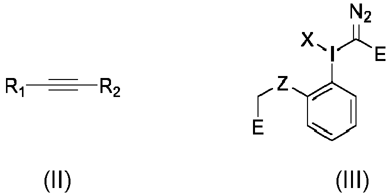 in the presence of a catalytically effective amount of a rhodium(II) catalyst, wherein X, R₁, R₂ and E are as defined in the first aspect of the invention; and wherein, in the compound of formula (III), X is attached to the iodine atom via a covalent or ionic bond and Z is a diradical selected from the group consisting of the diradicals of formula -COO-, -C((C₁-C₆)alkyl)₂O-, -SO₂-O-, -NR-O-, -B(OR)-O, -S-O-, and -P(O)(OR)-O-, wherein R is H or (C₁-C₆)alkyl.

The compound of the first aspect of the invention is useful in cyclopropenylation reaction. A third aspect of the invention thus relates to the use of a compound of formula (I) as defined in the first aspect of the invention as a cyclopropenylation reagent.

The compound of the first aspect of the invention is particularly useful as a cyclopropenylation reagent in electrophilic aromatic substitution reactions. A fourth aspect of the invention thus relates to a process for the preparation of a compound comprising a moiety of formula (IV) 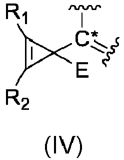 said process comprising the step of reacting a compound of formula (I) as defined in the first aspect of the invention with a compound comprising a moiety of formula (V) 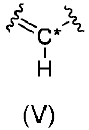 wherein C* represents a carbon atom comprised in an aromatic or heteroaromatic ring system and wherein said process converts the moiety of formula (V) in the moiety of formula (IV).

The compound of the first aspect of the invention is also particularly useful as a cyclopropenylation reagent of organometallic substrates. A fifth aspect of the invention relates to a process for the preparation of a compound comprising a moiety of formula (IX) 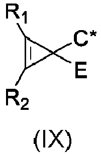
said process comprising the step of reacting a compound of formula (I) as defined in the first aspect of the invention with a compound comprising a moiety of formula C*-M, wherein C* represents a saturated or unsaturated carbon atom;
M represents a group selected from the group consisting of -MgX being X a halo group, a group of formula -B(OR₁₄)(OR₁₅) wherein R₁₄ and R₁₅ are selected from H and (C₁-C₆)alkyl or; alternatively, R₁₄ and R₁₅ together with the oxygen and boron atoms to which they are attached form a saturated ring optionally substituted at any available position with a (C₁-C₆)alkylgroup, and -ZnR₁₆ wherein R₁₆ is a (C₁-C₆)alkylgroup; and
wherein said process converts the moiety of formula (C*-M) in the moiety of formula (IX).

### BRIEF DESCRIPTION OF THE DRAWINGS

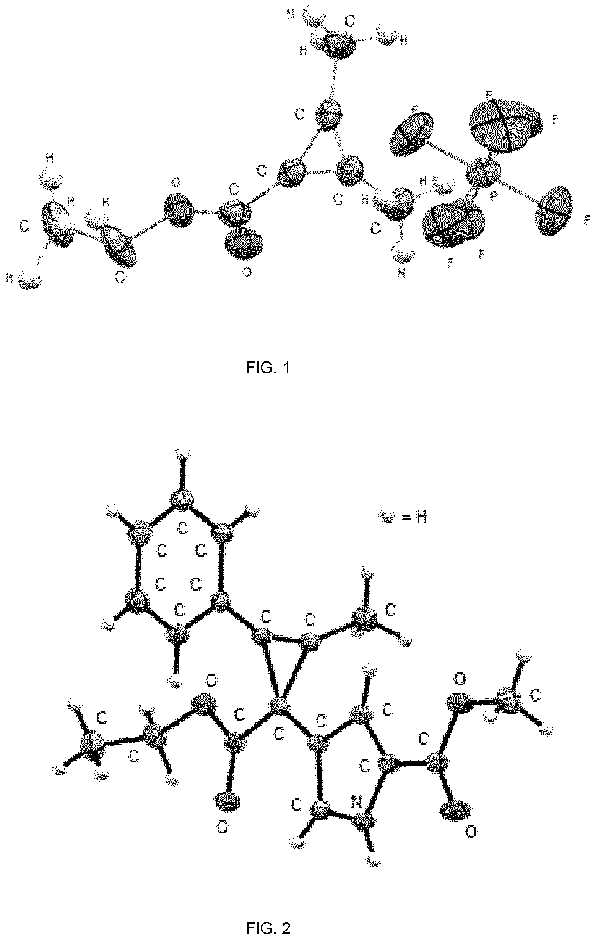

Fig. 1 shows the crystal structure as measured by X-Ray diffraction spectrometry of 1-(ethoxycarbonyl)-2,3-dimethylcyclopropenium hexafluorophosphate as prepared according to Example 2.
Fig. 2 shows the crystal structure as measured by X-Ray diffraction spectrometry of methyl 4-(1-(ethoxycarbonyl)-2-methyl-3-phenylcycloprop-2-en-1-yl)-1*H*-pyrrole-2-carboxylate as prepared according to Example 4.

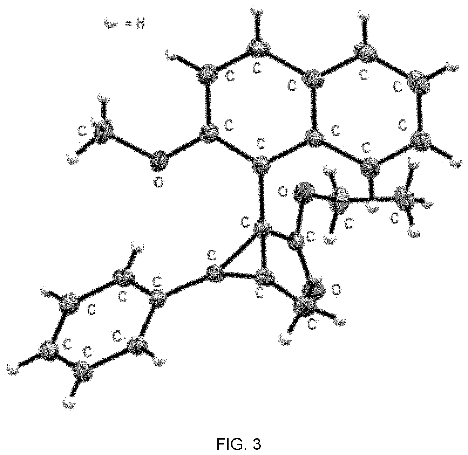

Fig. 3 shows the crystal structure as measured by X-Ray diffraction spectrometry of ethyl 1-(2-methoxynaphthalen-1-yl)-2-methyl-3-phenylcycloprop-2-ene-1-carboxylate as prepared according to Example 4.

### DETAILED DESCRIPTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the invention, any ranges given include both the lower and the upper end-points of the range. Ranges given, such as temperatures, times, molar ratio, volume ratio and the like, should be considered approximate (i.e. with a 5% margin of variation around indicated point), unless specifically stated.

Throughout the description and claims the term cyclopropenium refers to a trivalent triradical of formula C₃⁺. In particular, the compound of formula (I) of the first aspect of the invention may be depicted in any of the following tautomeric forms, all of which refer to the same compound: 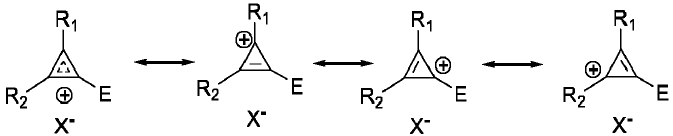 The compound of formula (I) exists as a mixture of the tautomeric forms depicted above. Depending on the electron donating nature or electron withdrawing nature of the R₁, R₂ and E groups in the compound of formula (I), a certain tautomeric form is favored over others. In particular embodiments of the first aspect of the invention, the following tautomeric form is favored, because E is an electron withdrawing group: 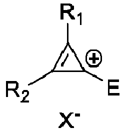 As described above, the existence of a tautomeric form favored over the other ones provides for regioselectivity when the compound of formula (I) is used as cyclopropenylation reagent.

In the context of the invention, the term "alkyl" refers to an aliphatic saturated hydrocarbon chain that is linear or branched and having the number of carbon atoms defined in the claims and the description. Non-limiting examples of alkyl groups include, for instance, methyl, ethyl, n-propyl, i-propyl, n-butyl, i-butyl, t-butyl, n-pentyl, neo-pentyl, hexyl, heptyl, octyl, nonyl, decyl, undecanyl and dodecanyl.

In the context of the invention, the term "haloalkyl" refers to an alkyl group as defined above and having the number of carbon atoms defined in the claims and the description whereby at least one of the hydrogen atoms is replaced with a halo group. The term "halo" group refers to a group selected from fluoro, chloro, bromo and iodo. Thus, non-limiting examples of haloalkyl groups include, among others, chloromethyl, fluoromethyl, perfluoroalkyl groups such as trifluoromethyl, tetrafluoroethyl, and bromomethyl.

In the context of the invention, the term "alkenyl" refers to an aliphatic unsaturated hydrocarbon chain that is linear or branched and having the number of carbon atoms defined in the claims and the description wherein at least two carbon atoms are linked through a double carbon-carbon bond. Non-limiting examples of alkenyl groups include, among others, vinyl, methylvinyl, propen-1-yl, propen-2-yl, butenyl, and iso-butenyl.

In the context of the invention, the term "alkylcarbonyl" refers to a saturated linear or branched hydrocarbon group having the number of carbon atoms indicated in the description or in the claims which is attached to the remainder of the molecular formula through a carbonyl group (C=O).

In the context of the invention, the term "alkylcarbonyloxy" refers to a saturated linear or branched hydrocarbon group having the number of carbon atoms indicated in the description or in the claims which is attached to the remainder of the molecular formula through a carboxyl group (-COO-) and wherein the alkyl chain is attached to the carbon atom of the carboxyl group.

In the context of the invention, the term "alkyloxycarbonyl" refers to a saturated linear or branched hydrocarbon group having the number of carbon atoms indicated in the description or in the claims which is attached to the remainder of the molecular formula through a carboxyl group (-OOC-) and wherein the alkyl chain is attached to the oxygen atom of the carboxyl group.

In the context of the invention, the term "alkenylcarbonyloxy" refers to an unsaturated linear or branched hydrocarbon group wherein at least two carbon atoms are linked through a double carbon-carbon bond and having the number of carbon atoms indicated in the description or in the claims which is attached to the remainder of the molecular formula through a carboxyl group (-COO-) and wherein the alkenyl chain is attached to the carbon atom of the carboxyl group.

In the context of the invention, the term "alkenyloxycarbonyl" refers to an unsaturated linear or branched hydrocarbon group wherein at least two carbon atoms are linked through a double carbon-carbon bond and having the number of carbon atoms indicated in the description or in the claims which is attached to the remainder of the molecular formula through a carboxyl group (- OOC-) and wherein the alkenyl chain is attached to the oxygen atom of the carboxyl group.

In the context of the invention, the term "alkylsulfonyl" refers to a saturated linear or branched hydrocarbon group having the number of carbon atoms indicated in the description or in the claims which is attached to the remainder of the molecular formula through a sulfoxy (-SO₂-) and wherein the alkyl chain is attached to the sulfur atom of the sulfoxy group.

In the context of the invention, the term "alkyloxysulfonyl" refers to a saturated linear or branched hydrocarbon group having the number of carbon atoms indicated in the description or in the claims which is attached to the remainder of the molecular formula through a diradical of formula -O-SO₂- and wherein the alkyl chain is attached to the oxygen atom of said diradical.

In the context of the invention, the term "electron withdrawing group" (also called EWG) refers to a group or molecular fragment able to withdraw electron density from the atom to which the group is attached, thereby polarizing the bond between the aforementioned atom and the EWG.

In the context of the invention, the term "alkyloxy" refers to a saturated linear or branched hydrocarbon group having the number of carbon atoms indicated in the description or in the claims which is attached to the remainder of the molecular formula through an oxy group (-O-).

In the context of the present invention, within a ring system comprising at least two rings, two rings are said to be "bridged" when they have at least two atoms in common in their cyclic structure, said atoms not being adjacent in either cyclic ring.

In the context of the present invention, within a ring system comprising at least two rings, two rings are said to be "fused" when they have at least two atoms in common in their cyclic structure, said atoms being adjacent in the cyclic rings.

In the context of the present invention, the term "aryl" or "aromatic" refers to a molecular fragment consisting of a conjugated planar ring system made of the number of carbon atoms defined in the description and in the claims, wherein the number of delocalized electrons is 4m+2, being m an integer number, being said system connected to the remainder parts of the compound comprising an aromatic ring system through any available position. Aromatic ring systems known in the art include, for instance, benzene, naphthalene, phenanthrene, indene, anthracene and tetracene.

In the context of the present invention, the term "heteroaryl" or "heteroaromatic" refers to a molecular fragment consisting of a conjugated planar ring system made of the number of carbon atoms defined in the description and in the claims and heteroatoms such as O, S, NH and N wherein the number of delocalized electrons is 4m+2, being m an integer number, being said system connected to the remainder parts of the compound comprising an aromatic ring system through any available position. Heteroaromatic ring systems known in the art include, for instance, furan, thiophene, pyridine, pyrazine, pyrimidine, pyrrole, imidazole, pyrazole, oxazole, isoxazole, thiazole, pyridazine, triazine, benzofuran, isobenzofuran, indole, isoindole, benzothiophene, benzo[c]thiophene, benzimidazole, purine, indazole, benzoxazole, benzisoxazole, benzothiazole, quinoline, isoquinoline, quinoxaline, acridine, quinazoline, cinnoline, and phthalazine.

In the context of the invention, the term "cycloalkyl" refers to a cyclic saturated hydrocarbon group having the number of carbon atoms indicated in the description or in the claims. Non-limiting examples of cycloalkyl include, among others, cyclopropyl, cyclobutyl, cyclopentyl, cyclohexyl, cycloheptyl and cyclooctyl.

In the context of the invention, the term "catalytically effective amount" refers to the fact that the amount of catalyst is much smaller than the stoichiometric amounts of either starting materials. The amount is expressed as percentage calculated as the ratio of the number of metal atoms comprised in the catalyst in relation to the number of molecules of compound of formula (II).

In the context of the invention, the term "cyclopropenylation" refers to a chemical reaction whereby a compound comprising a tetraradical of formula Y is formed, 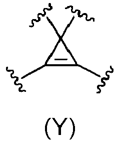 wherein the wavy lines indicate the attachment points of said tetraradical to the remainder of the compound, and wherein the cyclopropenylation reaction allows introducing at least one radical of formula (Y) in the starting material of the reaction.

In the context of the invention, the term "saturated carbon atom" refers to a carbon in sp³ hybridization state connected to four groups via single covalent bonds.

In the context of the invention, the term "unsaturated carbon atom" refers to a carbon in sp or sp² hybridization state connected to less than four groups via single covalent bonds and multiple covalent bonds.

As defined above, a first aspect of the invention relates to a compound of formula (I) as defined above in the first aspect of the invention.

In particular embodiments, the compound of formula (I) of the first aspect of the invention is one wherein E is an electron withdrawing group selected from the group consisting of (C₁-C₆)haloalkyl, a radical of formula -COOR₃, a formyl group (-CHO), (C₁-C₆)alkylcarbonyl, nitro and nitrile.

In more particular embodiments, the compound of formula (I) of the first aspect of the invention is one wherein E is an electron withdrawing group of formula -COOR₃.

In more particular embodiments, the compound of formula (I) of the first aspect of the invention is one wherein R₁ is selected from the group consisting of (C₁-C₁₂)alkyl, (C₂-C₁₂)alkenyl and a ring system comprising from 1 to 3 rings, each ring being saturated, unsaturated or aromatic, each ring comprising from 3 to 7 members selected from the group consisting of C, CH, CH₂, O, N, NH, NR₄ and S, each ring being isolated, bridged or fused; wherein each of the (C₁-C₁₂)alkyl, (C₂-C₁₂)alkenyl and said ring system is optionally substituted at any available position with one or more radicals selected from the group consisting of halogen, (C₆-C₂₀)aryl, (C₅-C₂₀)heteroaryl, (C₃-C₁₂)cycloalkyl, (C₁-C₆)alkyl, (C₁-C₆)haloalkyl, (C₁-C₆)alkyloxy, (C₁-C₆)alkylcarbonyl,(C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a formyl group, a nitro group, a radical of formula -PG₁, a radical of formula -OPG₁ and a radical of formula -NR₄R₅, wherein PG₁, R₄ and R₅ are as defined above.

In more particular embodiments, the compound of formula (I) of the first aspect of the invention is one wherein R₁ is selected from the group consisting of (C₁-C₁₂)alkyl, and a saturated, unsaturated or aromatic ring, said ring comprising from 3 to 7 members selected from the group consisting of C, CH, CH₂, O, N, NH, NR₄ and S; wherein each of the (C₁-C₁₂)alkyl and said ring system is optionally substituted at any available position with one or more radicals selected from the group consisting of halogen, (C₆-C₂₀)aryl, (C₅-C₂₀)heteroaryl, (C₃-C₁₂)cycloalkyl, (C₁-C₆)alkyl, (C₁-C₆)haloalkyl, (C₁-C₆)alkyloxy, (C₁-C₆)alkylcarbonyl,(C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a formyl group, a nitro group, a radical of formula -PG₁, a radical of formula -OPG₁ and a radical of formula -NR₄R₅, wherein PG₁, R₄ and R₅ are as defined above.

In more particular embodiments, the compound of formula (I) of the first aspect of the invention is one wherein R₁ is a (C₁-C₁₂)alkyl or a (C₃-C₈)cycloalkyl group, said (C₁-C₁₂)alkyl and (C₃-C₈)cycloalkyl groups being optionally substituted at any available position with one or more radicals selected from the group consisting of halogen, (C₆-C₂₀)aryl, (C₅-C₂₀)heteroaryl, (C₃-C₁₂)cycloalkyl, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a formyl group, a nitro group, a radical of formula -PG₁, a radical of formula -OPG₁ and a radical of formula -NR₄R₅.

In more particular embodiments, the compound of formula (I) of the first aspect of the invention is one wherein R₁ is a (C₁-C₁₂)alkyl or a (C₃-C₈)cycloalkyl group, said (C₁-C₁₂)alkyl and (C₃-C₈)cycloalkyl groups being optionally substituted at any available position with one or more radicals selected from the group consisting of halogen, (C₆-C₂₀)aryl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a nitro group, a radical of formula -PG₁, and a radical of formula -OPG₁.

In more particular embodiments, the compound of formula (I) of the first aspect of the invention is one wherein R₁ is selected from the group consisting of methyl, ethyl, n-propyl, *iso*-propyl, iso-butyl, chloromethyl, 4-chloro-*n*-butyl, benzyl, *N-*phthalimidylmethyl, 4-((tert-butyldiphenylsilyl)oxy)butyl, 4-((N-phthalimidyl)oxy)butyl, cyclopropyl, cyclobutyl, cyclohexyl and 2-phenylethyl.

In another particular embodiment, the compound of formula (I) of the first aspect of the invention is one wherein R₂ is selected from the group consisting of (C₁-C₁₂)alkyl, and a ring system comprising from 1 to 3 rings, each ring being saturated, unsaturated or aromatic, each ring comprising from 3 to 7 members selected from the group consisting of C, CH, CH₂, O, N, NH, NR₄ and S, each ring being isolated, bridged or fused; wherein each of the (C₁-C₁₂)alkyl, and said ring system is optionally substituted at any available position with one or more radicals selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a formyl group, and a nitro group, being R₄ as defined above. Preferably, when R₂ is a ring system, said ring system preferably comprises an aromatic ring.

In another particular embodiment, the compound of formula (I) of the first aspect of the invention is one wherein R₂ is selected from the group consisting of (C₁-C₁₂)alkyl and a ring system comprising from 1 to 2 aromatic rings, each ring comprising from 5 to 6 members selected from the group consisting of C, CH, O, N, NH, NR₄ and S, each ring being isolated or fused; said rings being further optionally substituted at any available position with one or more radicals selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, (C₂-C₆)alkenylcarbonyloxy and (C₂-C₆)alkenyloxycarbonyl, being R₄ as defined above.

In another particular embodiment, the compound of formula (I) of the first aspect of the invention is one wherein R₂ is selected from the group consisting of (C₁-C₁₂)alkyl and a ring system comprising from 1 to 2 aromatic rings, each ring comprising from 5 to 6 members selected from the group consisting of C, CH, O, N, NH, NR₄ and S, each ring being isolated or fused; said rings being further optionally substituted at any available position with one or more radicals selected from the group consisting of halogen, (C₁-C₆)alkyl, (C₁-C₆)haloalkyl, (C₁-C₆)alkylcarbonyloxy, (C₁-C₆)alkyloxycarbonyl, (C₂-C₆)alkenylcarbonyloxy and (C₂-C₆)alkenyloxycarbonyl, being R₄ as defined above.

Also preferably, when R₁, R₂ or R₃ is a ring system comprising at least a member that is NR₄, R₄ is selected from the group consisting of (C₁-C₆)alkyloxycarbonyl, benzyloxycarbonyl, 9-fluorenylmethoxycarbonyl; (C₁-C₆)alkylsulfonyl and phenylsulfonyl wherein the phenyl ring is optionally substituted with one or more radicals selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, nitrile, a formyl group and nitro.

In more particular embodiments, the compound of formula (I) of the first aspect of the invention is one wherein, when R₁, R₂ or R₃ is a ring system comprising at least a member that is NR₄, R₄ is selected from the group consisting of (C₁-C₆)alkyloxycarbonyl, benzyloxycarbonyl, 9-fluorenylmethoxycarbonyl; (C₁-C₆)alkylsulfonyl and phenylsulfonyl wherein the phenyl ring is optionally substituted with a (C₁-C₆)alkyl group.

In even more particular embodiments, the compound of formula (I) of the first aspect of the invention is one wherein, when R₁, R₂ or R₃ is a ring system comprising at least a member that is NR₄, R₄ is selected from the group consisting of *tert-*butyloxycarbonyl, benzyloxycarbonyl, 9-fluorenylmethoxycarbonyl; methylsulfonyl and p-tolylsulfonyl.

In even more particular embodiments, the compound of formula (I) of the first aspect of the invention is one wherein, when R₁, R₂ or R₃ is a ring system comprising at least a member that is NR₄, R₄ is *p*-tolylsulfonyl.

In another particular embodiment, the compound of formula (I) of the first aspect of the invention is one wherein R₂ is selected from the group consisting of methyl, 1-naphthyl, 2-naphthyl, *N*-(*p*-toluenesulfonyl)indol-5-yl, 4-phenylphenyl and phenyl optionally substituted with one or more radicals selected from the group consisting of halogen, trifluoromethyl, acetoxy, methyloxycarbonyl, vinylcarbonyloxy, methyl, ethyl and propyl.

In another particular embodiment, the compound of formula (I) of the first aspect of the invention is one wherein R₃ is selected from the group consisting of (C₁-C₁₂)alkyl and a ring system comprising from 1 to 2 rings, each ring being saturated, unsaturated or aromatic, each ring comprising from 3 to 7 members selected from the group consisting of C, CH, CH₂, O, N, NH, NR₄ and S, each ring being isolated, bridged or fused; wherein said ring system is optionally substituted at any available position with one or more radicals selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a formyl group, and a nitro group, being R₄ as defined above.

In another particular embodiment, the compound of formula (I) of the first aspect of the invention is one wherein R₃ is selected from the group consisting of (C₁-C₁₂)alkyl and a ring system comprising from 1 to 2 rings, each ring being saturated, unsaturated or aromatic, each ring comprising from 3 to 7 members selected from the group consisting of C, CH, CH₂, O, N, NH, NR₄ and S, each ring being isolated, bridged or fused; wherein said ring system is optionally substituted at any available position with a (C₁-C₆)alkyl group.

In another particular embodiment, the compound of formula (I) of the first aspect of the invention is one wherein R₃ is selected from the group consisting of (C₁-C₁₂)alkyl and a ring system comprising from 1 to 2 rings, each ring being saturated, unsaturated or aromatic, each ring comprising from 3 to 7 members selected from the group consisting of C, CH, and CH₂, each ring being isolated, bridged or fused; wherein said ring system is optionally substituted at any available position with a (C₁-C₆)alkyl group.

In another particular embodiment, the compound of formula (I) of the first aspect of the invention is one wherein R₃ is selected from the group consisting of (C₁-C₁₂)alkyl and a ring system comprising from 1 to 2 rings, each ring being saturated or aromatic, each ring comprising from 3 to 7 members selected from the group consisting of C, CH, and CH₂, each ring being isolated, bridged or fused; wherein said ring system is optionally substituted at any available position with a (C₁-C₆)alkyl group.

In another particular embodiment, the compound of formula (I) of the first aspect of the invention is one wherein R₃ is selected from the group consisting of methyl, ethyl, *iso*-propyl, phenyl and 2-*iso*-propyl-5-methyl-cyclohen-1-yl.

In another particular embodiment, the compound of formula (I) of the first aspect of the invention is one wherein Xⁿ⁻ is an anion selected from the group consisting of hexafluorophosphate, hexafluoroantimonate, perchlorate, trifluoromethanesulfonate, tetrafluoroborate and B(Ar)₄⁻ wherein Ar is a phenyl ring optionally substituted at any available position with a halogen group or a (C₁-C₆)haloalkyl group.

In another particular embodiment, the compound of formula (I) of the first aspect of the invention is one wherein xⁿ⁻ is an anion selected from the group consisting of hexafluorophosphate, hexafluoroantimonate, perchlorate trifluoromethanesulfonate and tetrafluoroborate.

In another particular embodiment, the compound of formula (I) of the first aspect of the invention is one wherein X⁻ is hexafluorophosphate.

The alternatives listed above for the groups of formulae E, R₁, R₂ and Xⁿ⁻ related to the compound of formula (I) may be combined - the resulting combinations also define preferred embodiments of the first aspect of the invention.

In particular, the compound of formula (I) is preferably one wherein:
R₁ is a (C₁-C₁₂)alkyl or a (C₃-C₈)cycloalkyl group, said (C₁-C₁₂)alkyl and (C₃-C₈)cycloalkyl groups being optionally substituted at any available position with one or more radicals selected from the group consisting of halogen, (C₆-C₂₀)aryl, (C₅-C₂₀)heteroaryl, (C₃-C₁₂)cycloalkyl, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a formyl group, a nitro group;
R₂ is selected from the group consisting of (C₁-C₁₂)alkyl and a ring system comprising from 1 to 2 aromatic rings, each ring comprising from 5 to 6 members selected from the group consisting of C, CH, O, N, NH, NR₄ and S, each ring being isolated or fused; said rings being further optionally substituted at any available position with one or more radicals selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl,
(C₂-C₆)alkenylcarbonyloxy and (C₂-C₆)alkenyloxycarbonyl; and E is a radical of formula -COOR₃ wherein R₃ is selected from the group consisting of (C₁-C₁₂)alkyl and a ring system comprising from 1 to 2 rings, each ring being saturated, unsaturated or aromatic, each ring comprising from 3 to 7 members selected from the group consisting of C, CH, CH₂, O, N, NH, NR₄ and S, each ring being isolated, bridged or fused; wherein said ring system is optionally substituted at any available position with a (C₁-C₆)alkyl group; being R₄ as defined above.

Thus, in more particular embodiments of the first aspect of the invention, the compound of formula (I) is selected from the group consisting of:
1-(ethoxycarbonyl)-2-phenyl-3-methylcyclopropenium hexafluorophosphate;
1-(ethoxycarbonyl)-2-(4-fluorophenyl)-3-methylcyclopropenium hexafluorophosphate;
1-(ethoxycarbonyl)-2-(4-chlorophenyl)-3-methylcyclopropenium hexafluorophosphate;
1-(ethoxycarbonyl)-2-(4-bromophenyl)-3-methylcyclopropenium hexafluorophosphate;
1-(ethoxycarbonyl)-2-(4-iodophenyl)-3-methylcyclopropenium hexafluorophosphate;
1-(ethoxycarbonyl)-2-(4-acetoxyphenyl)-3-methylcyclopropenium hexafluorophosphate;
1-(ethoxycarbonyl)-2-(4-phenyphenyl)-3-methylcyclopropenium hexafluorophosphate;
1-(ethoxycarbonyl)-2-(4-trifluorophenyl)-3-methylcyclopropenium hexafluorophosphate;
1-(ethoxycarbonyl)-2-(4-(methoxycarbonyl)phenyl)-3-methylcyclopropenium hexafluorophosphate;
1-(ethoxycarbonyl)-2-(4-acrylatephenyl)-3-methylcyclopropenium hexafluorophosphate;
1-(ethoxycarbonyl)-2-(3-methylphenyl)-3-methylcyclopropenium hexafluorophosphate;
1-(ethoxycarbonyl)-2-(3-bromophenyl)-3-methylcyclopropenium hexafluorophosphate;
1-(ethoxycarbonyl)-2-(2-chlorophenyl)-3-methylcyclopropenium hexafluorophosphate;
1-(ethoxycarbonyl)-2-(2-bromophenyl)-3-methylcyclopropenium hexafluorophosphate;
1-(ethoxycarbonyl)-2-(1-naphthalene)-3-methylcyclopropenium hexafluorophosphate;
1-(ethoxycarbonyl)-2-(2-naphthalene)-3-methylcyclopropenium hexafluorophosphate;
1-(ethoxycarbonyl)-2-(methyl)-3-(1-tosyl-1*H*-indol-5-yl)cyclopropenium hexafluorophosphate;
1-(ethoxycarbonyl)-2-phenyl-3-ethylcyclopropenium hexafluorophosphate;
1-(ethoxycarbonyl)-2-phenyl-3-(2-methyl)propylcyclopropenium hexafluorophosphate;
1-(ethoxycarbonyl)-2-phenyl-3-(chloromethyl)cyclopropenium hexafluorophosphate;
1-(ethoxycarbonyl)-2-phenyl-3-(4-chloro)butylcyclopropenium hexafluorophosphate;
1-(ethoxycarbonyl)-2-phenyl-3-benzylcyclopropenium hexafluorophosphate;
1-(ethoxycarbonyl)-2-phenyl-3-((*N*-phthalimidyl)methyl)cyclopropenium hexafluorophosphate;
1-(ethoxycarbonyl)-2-(4-((tert-butyldiphenylsilyl)oxy)butyl)-3-phenylcyclopropenium hexafluorophosphate;
1-(ethoxycarbonyl)-2-phenyl-3-(4-((1,3-dioxoisoindolin-2-yl)oxy)butyl)cyclopropenium hexafluorophosphate;
1-(ethoxycarbonyl)-2-phenyl-3-isopropylcyclopropenium hexafluorophosphate;
1-(ethoxycarbonyl)-2-phenyl-3-cyclopropylcyclopropenium hexafluorophosphate;
1-(ethoxycarbonyl)-2-phenyl-3-cyclobutylcyclopropenium hexafluorophosphate;
1-(ethoxycarbonyl)-2-phenyl-3-cyclohexylcyclopropenium hexafluorophosphate;
1-(ethoxycarbonyl)-2,3-dimethylcyclopropenium hexafluorophosphate;
1-(ethoxycarbonyl)-2,3-diethylcyclopropenium hexafluorophosphate;
1-(ethoxycarbonyl)-2-ethyl-3-methylcyclopropenium hexafluorophosphate;
1-(ethoxycarbonyl)-2-propyl-3-cyclopropylcyclopropenium hexafluorophosphate;
1-(ethoxycarbonyl)-2-benzyl-3-methylcyclopropenium hexafluorophosphate;
1-(ethoxycarbonyl)-2-(2-phenyl)ethyl-3-methylcyclopropenium hexafluorophosphate;
1-(methoxycarbonyl)-2-phenyl-3-methylcyclopropenium hexafluorophosphate;
1-(isopropoxycarbonyl)-2-phenyl-3-methylcyclopropenium hexafluorophosphate;
1-(phenoxycarbonyl)-2-phenyl-3-methylcyclopropenium hexafluorophosphate and
1-(2-isopropyl-5-methylcyclohexyloxycarbonyl)-2,3-diethylcyclopropenium hexafluorophosphate.

A process for the preparation of a compound of formula (I) is also part of the invention. As defined above, said process comprises the step of contacting a compound of formula (II) with a compound of formula (III) 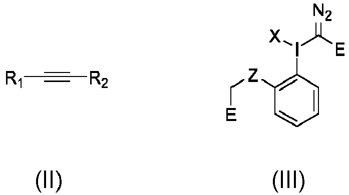 in the presence of a catalytically effective amount of a rhodium(II) catalyst, wherein X, R₁, R₂ and E are as defined in the first aspect of the invention; and wherein, in the compound of formula (III), X is attached to the iodine atom via a covalent or ionic bond and Z is a diradical selected from the group consisting of the diradicals of formula -COO-, -C((C₁-C₆)alkyl)₂O-, -SO₂-O-, -NR-O-, -B(OR)-O, -S-O-, and -P(O)(OR)-O-, wherein R is H or (C₁-C₆)alkyl.

Thus, in particular embodiments of the second aspect of the invention, R₁, R₂, E and xⁿ⁻ are independently as defined in any of the particular and preferred embodiments of the first aspect of the invention defined above.

In a particular embodiment of the second aspect of the invention, the compound of formula (III) is one wherein Z is a diradical selected from the group consisting of the diradicals of formula -COO-, -C((C₁-C₆)alkyl)₂O- and -SO₂-O-.

In a more particular embodiment of the second aspect of the invention, the compound of formula (III) is one wherein Z is a diradical of formula -COO-.

In a more particular embodiment of the second aspect of the invention, the rhodium(II) catalyst is one of formula (VII) 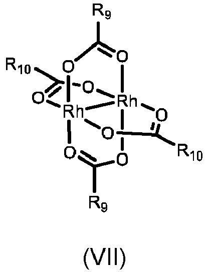
wherein each of Rg and R₁₀ is independently selected from the group consisting of (C₁-C₁₂)alkyl, (C₃-C₈)cycloalkyl and a radical of formula -CR₁₁R₁₂R₁₃ wherein each of R₁₁, R₁₂ and R₁₃ is independently selected from the group consisting of (C₁-C₁₂)alkyl, or, alternatively, wherein R₁₁, R₁₂ and R₁₃ form, together with the carbon atom to which they are attached a ring system comprising from 2 to 3 saturated hydrocarbon rings having from 3 to 8 members; or, alternatively,
wherein Rg and R₁₀, together with the carboxylate groups to which they are attached form a dicarboxylate compound of formula (VIII)

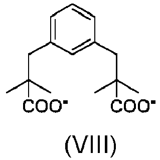

In a more particular embodiment of the second aspect of the invention, the rhodium(II) catalyst is one of formula (VII) as defined above wherein each of Rg and R₁₀ is independently selected from the group consisting of methyl, *tert*-butyl, heptyl, adamantyl; or, alternatively, wherein Rg and R₁₀, together with the carboxylate groups to which they are attached form a dicarboxylate compound of formula (VIII) as defined above.

In a more particular embodiment of the second aspect of the invention, the rhodium(II) catalyst is selected from the group consisting of the compounds of formulae Rh₂(OCOCH₃)₄, Rh₂(OCOt-Bu)₄, Rh₂(OCOC₇H₁₅)₄, Rh₂(OCOAd)₄ whereby Ad represents an adamantyl group and Rh₂(esp)₂, represented by the fomula:

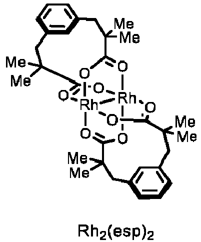

In a more particular embodiment of the second aspect of the invention, the rhodium(II) catalyst is Rh₂(esp)₂ as defined above. The compound of formula Rh₂(esp)₂ may also be referred to herein as bis[rhodium(α,α,α',α'-tetramethyl-1,3-benzenedipropionic acid)].

In a more particular embodiment of the second aspect of the invention, the rhodium(II) catalyst is present in an amount of between 0.1 mol% and 2 mol%. Preferably, it is present in an amount of between 1 mol% and 2 mol%. In a more particular embodiment of the second aspect of the invention, the rhodium(II) catalyst is present in an amount of in an amount of 1 mole of catalyst per each 100 moles of compound of formula (III), that is 1 mol%.

In a more particular embodiment of the second aspect of the invention, the molar ratio of the compound of formula (II) to the compound of formula (III) is of from 1:2 to 5:1.

In a more particular embodiment of the second aspect of the invention, the molar ratio of the compound of formula (II) to the compound of formula (III) is of from 1:1 to 3:1. In a more particular embodiment of the second aspect of the invention, the molar ratio of the compound of formula (II) to the compound of formula (III) is of from 1.3:1 to 2:1.

In a more particular embodiment of the second aspect of the invention, the process is carried out at a temperature of below - 40 °C. This advantageously prevents degradation of the formed cyclopropenium cation.

In a more particular embodiment of the second aspect of the invention, the process is carried out at a temperature of below - 50 °C; more particularly of about - 60 °C.

In a more particular embodiment of the second aspect of the invention, the process is carried out in a chlorinated organic solvent. Suitable chlorinated solvents include dichloromethane (DCM), chloroform, 1,2-dichloroethane (DCE), carbon tetrachloride (CCl₄), chlorobenzene, 1,2-dichlorobenzene, 1,1,1-trichloroethane and trichloroethylene.

In a more particular embodiment of the second aspect of the invention, the process is carried out in dichloromethane.

In a more particular embodiment of the second aspect of the invention, the process of the second aspect of the invention comprises the step of isolating the compound of formula (I).

Said isolation step is preferably carried out by precipitation of the compound of formula (I) from the reaction mixture. Preferably, the compound of formula (I) is precipitated from the reaction mixture by precipitation from a (C₄-C₇)alkane solvent. Said solvent may be added to the reaction mixture directly. It is further preferred that the formed precipitate is filtered in a minimum amount of time.

In a more particular embodiment of the second aspect of the invention, the process satisfies at least one, preferably two or more, of the conditions (i) to (vi):
(i) the rhodium(II) catalyst is bis[rhodium(α,α,α',α'-tetramethyl-1,3-benzenedipropionic acid)];
(ii) the catalyst is present in an amount of 1 mole of catalyst per each 100 moles of compound of formula (III);
(iii) the molar ratio of the compound of formula (II) to the compound of formula (III) is of from 1:1 to 3:1;
(iv) the process is carried out at a temperature of below - 40 °C;
(v) the process is carried out in a chlorinated organic solvent; and
(vi) the compound of formula (I) is isolated by precipitation from a (C₄-C₇)alkane solvent.

In a more particular embodiment of the second aspect of the invention, the process satisfies all the conditions (i) to (vi) defined above.

As mentioned above, a third aspect of the invention relates to the use of the compound according to the first aspect of the invention as cyclopropenylation reagent. As referred to herein, a "cyclopropenylation reagent" refers to a compound suitable for transferring a cyclopropenyl group to a reaction substrate. As mentioned above, the compound of formula (I) is such that the E group is an electron withdrawing group and have more electron withdrawing character than the R₁ and R₂ groups. When the compound of formula (I) is used as cyclopropenylation reagent, this advantageously provide the formation of a cyclopropenylated product with high regioselectivity, because the carbon atom adjacent to the electron withdrawing group is less electron rich than the carbon atoms bearing R₁ and R₂, and is thus more prompt to react with nucleophiles.

Consequently, when used as a cyclopropenylation reagent, the compound of formula (I) allows forming a product comprising the cyclopropenyl radical of formula with high regioselectivity: 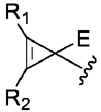 whereby the wavy line represents the attachment to the remainder of the molecule. The term "high regioselectivity" refers to the fact that the regioisomer product is formed in higher amounts than the other possible regioisomers. In particular, it is formed with a percentage higher than 60%. More particularly, said regioisomer is formed with a percentage higher than 75%; more particularly higher than 90% and even more particularly, higher than 95%.

Additionally, the cyclopropenium cation is known in the art for being highly reactive to a broad range of nucleophiles. The presence of an electron withdrawing substituent E on the cyclopropenium ring further enhances the reactivity towards nucleophiles.

Thus, in particular embodiments of the third aspect of the invention, the compound of formula (I) is used in the cyclopropenylation of nucleophilic substrates. Suitable nucleophilic substrates are known in the art and include, among others, alcohols, amines, thiols, organoboron compounds and organometallic reagents such as Grignard reagent, organotin reagents, organozinc reagents and organolithium reagents.

In such organometallic reagents, the metal atom may be attached to a carbon atom forming part of an aromatic or heteroaromatic ring, an alkyl group, a vinyl group or an allyl group; said groups forming part of the nucleophile.

As mentioned above and detailed in the fourth aspect of the invention, it is further preferred that the nucleophile is a compound comprising an aromatic or heteroaromatic moiety, whereby an aromatic carbon atom may act as a nucleophile.

Thus, a fourth aspect of the invention relates to a process for the preparation of a compound comprising a moiety of formula (IV) as defined above; said process comprising the step of reacting a compound of formula (I) as defined in the first aspect of the invention with a compound comprising a moiety of formula (V) as defined above wherein said process converts the moiety of formula (V) in the moiety of formula (IV).

In a particular embodiment of the fourth aspect of the invention, the compound of formula (I) is as defined in any of the particular and preferred embodiments defined above for the first aspect of the invention.

In a particular embodiment of the fourth aspect of the invention, the compound of formula (I) is selected from the group consisting of 1-(ethoxycarbonyl)-2-phenyl-3-methylcyclopropenium hexafluorophosphate, 1-(ethoxycarbonyl)-2-(4-trifluorophenyl)-3-methylcyclopropenium hexafluorophosphate and 1-(ethoxycarbonyl)-2,3-dimethylcyclopropenium hexafluorophosphate.

In a more particular embodiment of the fourth aspect of the invention, the moiety of formula (V) is comprised in a moiety deriving from an aromatic or heteroaromatic ring system selected from the group consisting of benzene, tetrahydronaphthalene, naphthalene, anthracene, indole, pyrrole, thiophene, benzothiophene, benzofuran, quinoline, naphtyridine, imidazole, thiazole, carbazole and pyridine.

In a more particular embodiment of the fourth aspect of the invention, the moiety of formula (V) is comprised in a moiety deriving from an aromatic or heteroaromatic ring system selected from the group consisting of benzene, tetrahydronaphthalene, naphthalene, indole, pyrrole, thiophene, dihydrobenzofuran, pyrimidine and pyridine.

In another particular embodiment of the fourth aspect of the invention, the compound comprising the moiety of formula (V) is selected from the group consisting of phenol, anisole, 4-methyl-1,1'-biphenyl, toluene, 4-fluorobenzene, benzene, p-xylene, m-xylene, o-xylene, 1-(tert-butyl)-4-methylbenzene, 1-fluoro-3-methylbenzene, 2-methoxybenzoate, 6-methoxy-1,2,3,4-tetrahydronaphthalene, *tert-*butyl(methyl)phenylcarbamate, mesitylene, naphthalene, 2-methoxynaphthalene, 1-methoxynaphthalene, 1-methyl-1h-indole, methyl 1h-pyrrole-2-carboxylate, 2-phenylthiophene, 6,7-dihydrobenzofuran-4(5*H*)-one, 2,3-dihydrobenzofuran, 2,6-dimethoxypyridine, indomethacin methyl ester, estragole, carbofuran, chlorpropham, ciprofibrate methyl ester, desipramine hydrochloride, propranolol, gemfibrozil, clopidrogrel, aripiprazole, pyriproxyfen, estradiol dimethyl ether, estrone, paroxetine hydrochloride, lopinavir, (S)-naproxen methyl ester, 5,5-difluoro-1,3,7,9-tetramethyl-5*H-*4λ4,5λ4-dipyrrolo[1,2-c:2',1'-f][1,3,2]diazaborinine and 5,5-difluoro-1,3,7,9,10-pentamethyl-5*H*-**4λ4,5λ4**-dipyrrolo[1,2-*c*:2',1'-*f*][1,3,2]diazaborinine.

In a more particular embodiment of the fourth aspect of the invention, the process is carried out in a solvent. Preferably, said solvent is selected from the group consisting of methanol, ethanol, isopropanol, hexafluoroisopropanol, trifluoroethanol and a chlorinated solvent. Suitable chlorinated solvents are dichloromethane (DCM), chloroform, 1,2-dichloroethane (DCE), carbon tetrachloride (CCl₄), chlorobenzene, 1,2-dichlorobenzene, 1,1,1-trichloroethane, and trichloroethylene.

In a more particular embodiment of the fourth aspect of the invention, the process is carried out in a solvent selected from the group consisting of hexafluoroisopropanol, trifluoroethanol and dichloromethane (DCM). More preferably, the process is carried out in a solvent that is hexafluoroisopropanol.

In a more particular embodiment of the fourth aspect of the invention, the process is carried out in the presence of an additive. Such additive may be a base, provided that it is not nucleophilic. Such bases are for instance alkaline salts of fluoride or bromide, alkaline salts of carbonate or hydrogen carbonate. Alternatively, the additive may be a molecular sieves or a crown ether compound, such as 15-C-5.

In a more particular embodiment of the fourth aspect of the invention, the process is carried out in the presence of an additive that is preferably caesium fluoride.

In another particular embodiment of the fourth aspect of the invention, the process is carried out in the presence of an additive and said additive is present in an amount of at least 1 mole of additive per each mole of the compound comprising the moiety of formula (V).

In another particular embodiment of the fourth aspect of the invention, the process is carried out in the presence of an additive and said additive is present in an amount of from 1 mole to 2 moles of additive per each mole of the compound comprising the moiety of formula (V).

In another particular embodiment of the fourth aspect of the invention, the process is carried out in the presence of an additive and said additive is present in an amount of 1.5 mole per each mole of the compound comprising the moiety of formula (V).

In another particular embodiment of the fourth aspect of the invention, the process satisfies at least one, preferably 2 or 3, of the conditions (i) to (iii):
(a) the process is carried out in a solvent selected from the group consisting of dichloromethane, hexafluoroisopropanol and trifluoroethanol; preferably, the solvent is hexafluoroisopropanol;
(b) the process is carried out in the presence of an additive selected from the group consisting of alkaline salts of hydrogen carbonate, alkaline salts of fluoride, alkaline salts of carbonate, crown ether 15-C-5 and molecular sieves; preferably, the additive is caesium fluoride; and
(c) when the process is carried out in the presence of an additive as defined in (ii), said additive is present in an amount of 1.5 mole per each mole of the compound comprising the moiety of formula (V).

The process of the fourth aspect of the invention may comprise additional synthetic steps for modifying the formed cyclopropenyl derivative. Such modification steps can be implemented according to procedures known to the skilled person and which are common in the art.

Thus, in a more particular embodiment, the process of the fourth aspect of the invention comprises a further step of modifying the cyclopropenyl moiety of formula (IV) by submitting the reaction product to reactions selected from the group consisting of reduction, cycloaddition reaction, rhodium-catalyzed hydrothiolation, decarboxylation, hydrostannation, hydroborylation, intermolecular Pauson-Khand reaction, reaction with tetrazine derivatives and cycloisomerization (e.g. to furan derivative when E is COOR₃).

The skilled person will readily identify conditions of reaction suitable for carrying out such transformations. For instance, the reduction of the cyclopropenyl ring to a cyclopropyl ring may be undertaken by catalytic hydrogenation or reduction with ahydride.

The high reactivity of the cyclopropenyl moiety of formula (IV) in a broad range of conditions is advantageous, as it allows creating molecular diversity from a common synthetic precursor. In addition, the specific reaction of cyclopropenyl derivatives with phenyl or diphenyl tetrazine derivatives provides a strategy to use the cyclopropenyl ring as a chemical reporter, as disclosed in J. Am. Chem. Soc. 2012, 134, 45, 18638-18643.

As defined above, a fifth aspect of the invention relates to a process for the preparation of a compound comprising a moiety of formula (IX) 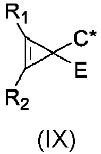
said process comprising the step of reacting a compound of formula (I) as defined in any one of the preferred and particular embodiments of the first aspect of the invention with a compound comprising a moiety of formula C*-M,
wherein C* represents a saturated or unsaturated carbon atom;
M represents a group selected from the group consisting of -Li, -MgX being X a halo group, a group of formula -B(OR₁₄)(OR₁₅) wherein R₁₄ and R₁₅ are selected from H and (C₁-C₆)alkyl or; alternatively, R₁₄ and R₁₅ together with the oxygen and boron atoms to which they are attached form a saturated ring optionally substituted at any available position with a (C₁-C₆)alkyl group, and -ZnR₁₆ wherein R₁₆ is a (C₁-C₆)alkyl group; and wherein said process converts the moiety of formula (C*-M) in the moiety of formula (IX).

In preferred embodiments of the fifth aspect of the invention, the process is carried out in a chlorinated solvent, such as dichloromethane.

In preferred embodiments of the fifth aspect of the invention, M represents a group selected from the group consisting of -MgX being X a chloro or bromo group, a group of formula -B(OR₁₄)(OR₁₅) wherein R₁₄ and R₁₅ are H or; alternatively, R₁₄ and R₁₅ together with the oxygen form a pinacol group, and and -ZnR₁₆ wherein R₁₆ is a (C₁-C₆)alkyl group.

In more preferred embodiments of the fifth aspect of the invention, M represents a group selected from the group consisting of -MgX being X a chloro or bromo group, a group of formula -B(OR₁₄)(OR₁₅) wherein R₁₄ and R₁₅ are H or; alternatively, R₁₄ and R₁₅ together with the oxygen form a pinacol group, and -ZnR₁₆ wherein R₁₆ is an ethyl group.

In other preferred embodiments of the fifth aspect of the invention, C* is comprised in an alkyl chain or or alkenyl chain.

In other preferred embodiments of the fifth aspect of the invention, C* is comprised in an aromatic or heteroaromatic ring.

In other preferred embodiments of the fifth aspect of the invention, the compound comprising a moiety of formula (IX) is selected from the group consisting of phenyl magnesium chloride, methyl magnesium bromide, vinyl magnesium bromide, 4-fluorophenylmagnesium bromide, 4-methoxyphenylmagnesium bromide, p-tolylmagnesium bromide, 4-chlorophenylmagnesium bromide, 3,5-bis(trifluoromethyl)phenylmagnesium bromide, 3-methoxyphenylmagnesium bromide, benzylmagnesium bromide, diethylzinc, naphthalen-1-ylboronic acid, p-tolylboronic acid and p-tolylboronic acid pinacol ester.

Throughout the description and claims the word "comprises" and variations of the word, are not intended to exclude other technical features, additives, components or steps. Furthermore, the word "comprise" encompasses the cases of "consist of" and "consists essentially of". Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### General considerations

All reagents were used as purchased and used with no further purification. Ethyl diazoacetate, (≥13 wt. % dichloromethane) was purchased from Aldrich (Ref. E22201) and used without further purification. Anhydrous solvents were dried by passing through an activated alumina column on a PureSolv^{™} solvent purification system (Innovative Technologies, Inc., MA). Analytical thin layer chromatography (TLC) was carried out using aluminum sheets with 0.2 mm of silica gel (Merck GF234). Visualization of the developed chromatogram was performed by irradiation with UV light or treatment with a solution of potassium permanganate or vanillin stain followed by heating. Flash column chromatography was performed on silica gel (Aldrich, 230-400 mesh) or neutral silica gel (Material Harvest Ltd., 230-400 mesh). Organic solutions were concentrated under reduced pressure on a Büchi rotatory evaporator. Unless otherwise stated, reactions were carried out under argon atmosphere. Yields refer to purified compounds unless otherwise noted. NMR spectra were recorded at 298 K on Bruker Avance 300, Bruker Avance 400 Ultrashield or Bruker Avance 500 Ultrashield apparatuses. Melting points were measured using open glass capillaries in a Büchi B540 apparatus. Infrared spectra were recorded on a Bruker Tensor 27. Mass spectra were recorded on a Waters LCT Premier spectrometer. Liquid chromatography-mass spectrometry (LC-MS) and gas chromatography-mass spectrometry (GC-MS) analysis were carried out in Agilent 1260 Infinity - 6130 Quadrupole and Agilent 7890B - 5977A MSD, respectively. X-Ray diffraction analysis were carried out in Rigaku MicroMax-007HF with pilatus 200K single crystal diffractometer and Bruker Apex II Duo single crystal diffractometer.

### Preparative Example 1: Preparation of alkyne compounds

The following non-terminal alkyne compounds were prepared or purchased:

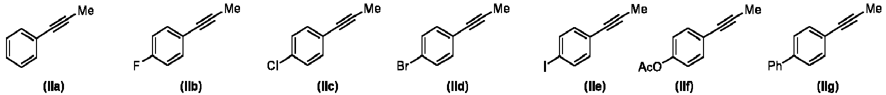

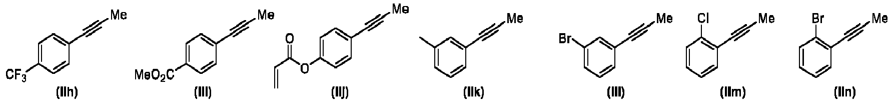

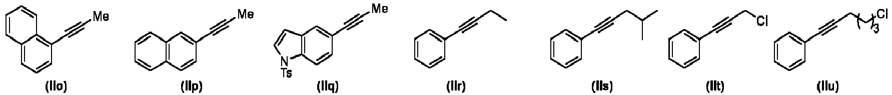

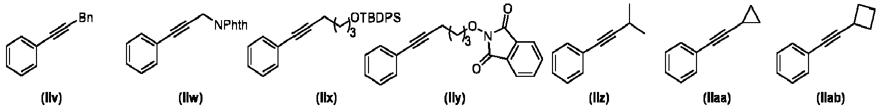

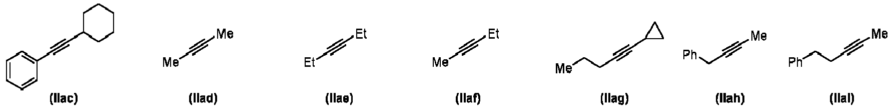

The compounds of formula (IIa), (IIr), (IIt), (IIad), (IIae) and (IIaf) are commercially available and have been used directly as received.

The compounds of formulae (IIb)-(IId), (IID-(IIp), (IIs)-(IIz), (IIaa-(IIac) were prepared according to the procedure disclosed in Gao, S. et al. Green Chem. 2017, 19, 1861-1865 (incorporated herein by reference) or in Berthold, D. et al. Org. Lett. 2018, 20, 598-601 (incorporated herein by reference) by the Sonogashira reaction between the corresponding iodo/bromoarene and terminal alkynes compounds.

(IIe) was synthesized from 4-iodobenzaldehyde according to the procedure disclosed in An, D.-L. et al. Synlett 2007, 12, 1909-1912 (incorporated herein by reference). (IIq) was synthesized by following a reported protocol based on a decarboxylative cross-coupling reaction disclosed in Cruz, F. A. et al. J. Am. Chem. Soc. 2017, 139, 1029-1032 (incorporated herein by reference). (IIag), (IIah), (IIai) were prepared by alkylation of the corresponding lithium acetylide derived from cyclopropylacetylene, 1-phenyl-2-propyne and 4-phenyl-1-butyne based on a procedure disclosed in Deponti, M. Org. Biomol. Chem. 2013, 11, 142-148 (incorporated herein by reference) or in Tan, E. H. P Angew. Chem. Int. Ed. 2011, 50, 9602-9606 (incorporated herein by reference).

### Preparative Example 2: Preparation of hypervalent iodine reagents

The following hypervalent iodine compounds were prepared:

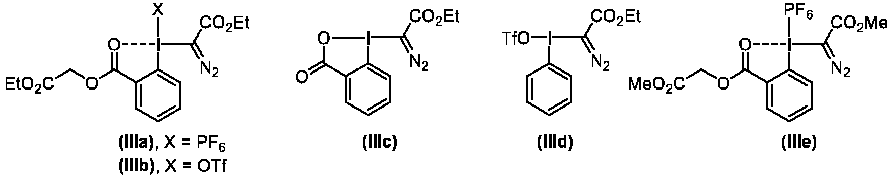

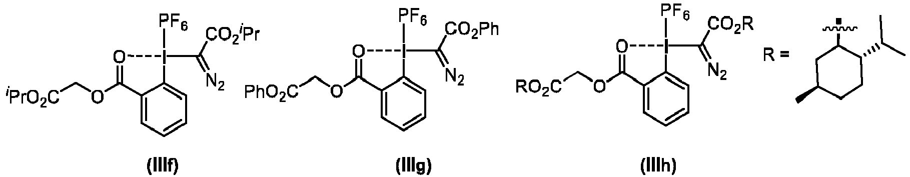

The compounds of formulae (IIIa)-(IIId) are known in the art, while compounds (IIIe) to (IIIh) are not known. These compounds were prepared by adapting the procedures described in Wang, Z. et al. J. Am. Chem. Soc. 2019, 141, 15509-15514 (incorporated herein by reference) or in Jiang, L. et al. Chem. Sci. 2022. DOI: 10.1039/D2SC00968D (incorporated herein by reference) and following the following general procedure:

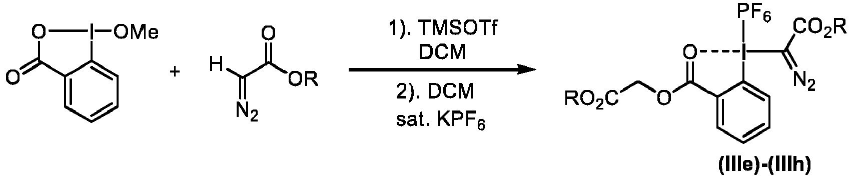

A solution of 1-methoxy-1,2-benziodoxol-3(1*H*)-one (1.0 equiv.) in dichloromethane (0.5 M) was treated with trimethylsilyl trifluoromethanesulfonate (1.0 equiv.) at room temperature. After 30 minutes, a cloudy suspension was observed and the corresponding diazo compound (2.2 equiv.) was added dropwise during 10 minutes. Nitrogen evolution was observed and the resulting reaction mixture was stirred at room temperature until a clear yellow solution was observed (1 hour). Solvent was removed under vacuum and the crude was recrystallized from a mixture of diethyl ether/dichloromethane (5/1) during 12 hours at-20 °C. The desired product (III)-OTf was collected by filtration, washed with cold diethyl ether, dried under high vacuum and stored at -20 °C. A solution of (III)-OTf in dichloromethane (0.2 M) was added to a separation funnel and then washed with a saturated aqueous solution of KPF₆. The combined organic layers were dried over Na₂SO₄ and solvent was removed under vacuum to give (IIIe)-(IIIh) as yellow solid.

(IIIe): HRMS (MALDI): calculated for C₁₃H₁₂IN₂O₆ [M-PF₆]+ m/z: 418.9740, found: 418.9739. IR v max (film, cm-1): 2123, 1707, 1659, 1557, 1437, 1384, 1278, 1151, 837, 745.

(IIIf): HRMS (MALDI): calculated for C₁₇H₂₀IN₂O₆[M-PF₆]⁺ m/z: 475.0366, found: 475.0380. IR v max (film, cm⁻¹): 2119, 1699, 1657, 1588, 1428, 1386, 1323, 1270, 1223, 1151, 1099, 1028, 827.

(IIIg): HRMS (MALDI): calculated for C₂₃H₁₆IN₂O₆[M-PF₆]⁺ m/z: 543.0053, found: 543.0077. IR v max (film, cm⁻¹): 2121, 1772, 1717, 1654, 1587, 1425, 1383, 1328, 1262, 1185, 1147, 829, 769.

(IIIh): HRMS (MALDI): calculated for C₃₁H₄₄IN₂O₆[M-PF₆]⁺ m/z: 667.2244, found: 667.2247. IR v max (film, cm⁻¹): 2954, 2926, 2868, 2121, 1751, 1700, 1662, 1220, 1026, 746.

### Example 1: Preparation of cyclopropenium cations of formula (I): reaction optimization and scope

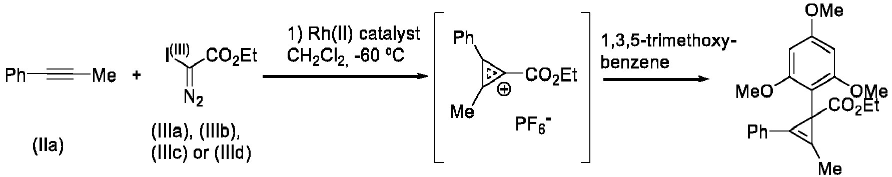

### General Procedure for reaction optimization:

To a 10 mL oven-dried tube equipped with a stirring bar was added the corresponding Rh catalyst as indicated in Table 1 (0.001 mmol, 1 mol%). The tube was sealed before being evacuated and backfilled with argon. 1-Phenyl-1-propyne (IIa) (23.2 mg, 25 µL, 0.2 mmol) and degassed dichloromethane (0.5 mL) were added and the resulting mixture was placed into a cooling bath at -60 °C. Then, a solution of the corresponding reagent of formula (III) as indicated in Table 1 (0.1 mmol, 1.0 equiv.) in degassed dichloromethane (1.0 mL) was added dropwise during 40 min using a syringe pump and a precipitate was generally observed. The reaction mixture was stirred for additional 10 min at -60 °C. After this, 1,3,5-trimethoxybenzene (0.4 mmol, 4.0 equiv.) was added as solid at -60 °C and the precipitate disappeared almost instantaneously. The reaction mixture was then quenched with triethylamine (0.5 mL) and the solvent was removed under *vacuum.* The resulting reaction crude mixture was analyzed by ¹H NMR using dibromomethane as internal standard to determine the yield of the reaction.

The rhodium(II) catalysts shown in Table 1 below are:

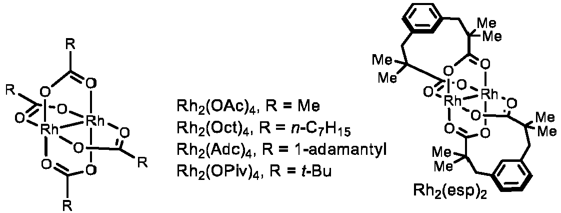

Table 1 shows the results obtained for said screening experiments.

**Table 1**

| alkyne (IIa) (equiv.) | Compound (III) | Rh catalyst | NMR yield 3a' (%) |
|---|---|---|---|
| 2.0 | (IIIa) | Rh₂(OAc)₄ | 4 |
| 2.0 | (IIIa) | Rh₂(OPiv)₄ | 93 |
| 2.0 | (IIIa) | Rh₂(Oct)₄ | 76 |
| 2.0 | (IIIa) | Rh₂(esp)₂ | >99 |
| 2.0 | (IIIa) | Rh₂(Adc)₄ | 90 |
| 2.0 | (IIIb) | Rh₂(esp)₂ | 50 |
| 2.0 | (IIIc) | Rh₂(esp)₂ | 0 |
| 2.0 | (IIId) | Rh₂(esp)₂ | 0 |
| 2.0 | (IIIa) | Rh₂(esp)₂ | 81^{a} |
| 2.0 | (IIIb) | Rh₂(esp)₂ | 14^{a} |
| 1.5 | (IIIa) | Rh₂(esp)₂ | >99 |
| 1.3 | (IIIa) | Rh₂(esp)₂ | >99 |
| 1.1 | (IIIa) | Rh₂(esp)₂ | 94 |
| 2.0 | (IIIa) | Rh₂(esp)₂ | 38*^{b}* |
| 2.0 | (IIIc) | Rh₂(esp)₂ | 60*^{c}* |

| | | | |
|---|---|---|---|
| *^{a}* Reaction carried out at -50 °C. *^{b}* After the addition of (IIa), the reaction mixture was allowed to warm to room temperature during 2 hours. *^{c}*0.5 equiv. of Zn(OTf)₂ was added. | | | |

As will be obvious to the skilled person, entries where the compound of formula (III) is (IIIc) or (IIId) are provided as comparative examples because these compounds do not fall under the scope of the second aspect of the invention.

The results of Table 1 suggest that the formation of cyclopropenium salts from a non-terminal alkyne, such as the compound of formula (IIa) and hypervalent iodine reagents of formula (III) as defined in the second aspect of the invention takes place in the presence of a rhodium(II) catalyst. Results show that said catalyst may be a rhodium tetracarboxylate compound whereby the carbon atom adjacent to the carboxylate functional group is a tertiary carbon atom forming part of an adamantyl group or part of a (C₁-C₁₂)alkyl chain. A preferred catalyst is Rh₂(esp)₂ as defined above.

### Example 2: Preparation of cyclopropenium salts of formula (I)

Table 2 below summarizes the results obtained for the preparation of cyclopropenium salts of formula (I) according to the general reaction scheme

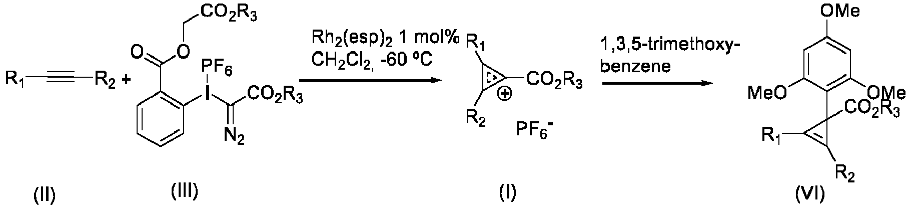

Said compounds may be prepared following the following general procedure:

To a 10 mL oven-dried tube equipped with a stirring bar was added Rh₂(esp)₂ (2.0 mg, 0.002 mmol, 1 mol%). The tube was sealed before being evacuated and backfilled with argon. The corresponding alkyne of formula (II) indicated in Table 2 (0.26 mmol, 1.3 equiv.) and degassed dichloromethane (0.5 mL) were added and the resulting mixture was cooled to -60 °C. Then, a solution of reagent of formula (III) indicated in Table 2 (0.20 mmol, 1.0 equiv.) in degassed dichloromethane (2.0 mL) was added dropwise during 40 min using a syringe pump. The reaction mixture was stirred for additional 10 min at -60 °C. After this, dry hexane (3.0 mL) was added slowly to the reaction mixture and the precipitate of compound of formula (I) was collected by filtration, washed with dry hexane (2.0 mL x 3) and dried under vacuum at room temperature.

In some cases, the precipitate of formula (I) was not observed probably due to a higher solubility of the corresponding cyclopropenium compound. In such cases, the aforementioned procedure was modified as follows: After the solution of reagent of formula (III) in degassed dichloromethane was added dropwise, 1,3,5-trimethoxybenzene (0.8 mmol, 4.0 equiv.) was added as solid at -60 °C. Then, the reaction mixture was quenched with triethylamine (0.5 mL) and the solvent was removed under vacuum. The crude residue was purified by flash column chromatography on neutral silica gel to give the corresponding compound of formula (VI). Table 3 provides the correspondences of the product number with the product name.

**Table 2**

| R₁ | R2 | R₃ | Product number | Yield (%) | HR-MS (g/mol) |
|---|---|---|---|---|---|
| Ph | Me | Et | (Ia) | 86 | 201.0909 |
| Ph | Me | Et | (Via) | 94 | 391.1512. |
| 4-fluorophenyl | Me | Et | (Ib) | 81 | 219.0815 |
| 4-chlorophenyl | Me | Et | (Ic) | 67 | 235.0530 |
| 4-bromophenyl | Me | Et | (Id) | 73 | 279.0006 |
| 4-iodophenyl | Me | Et | (le) | 68 | 326.9861 |
| 4-acetyl | Me | Et | (If) | 67 | 259.0953 |
| 4-phenylphenyl | Me | Et | (Ig) | 63 | 277.1223 |
| 4-CF₃-phenyl | Me | Et | (Ih) | 63 | 269.0785 |
| 4-CO₂Me-phenyl | Me | Et | (Ii) | 60 | 259.0959 |
| 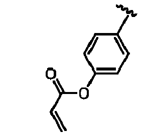 | Me | Et | (Ij) | 56 | 271.0962 |
| 3-methylphenyl | Me | Et | (Ik) | 59 | 215.1070 |
| 3-bromophenyl | Me | Et | (II) | 59 | 279.0017 |
| 2-chlorophenyl | Me | Et | (Im) | 57 | 235.0514 |
| 2-bromophenyl | Me | Et | (In) | 76 | 279.0016 |
| Naphth-1-yl | Me | Et | (Io) | 79 | 251.1072 |
| Naphth-2-yl | Me | Et | (Ip) | 68 | 251.1064 |
| 1-tosyl-1*H*-indol-5-yl | Me | Et | (Vlq) | 54 | 584.1708 |
| Phenyl | Et | Et | (Ir) | 68 | 215.1065 |
| Phenyl | Isobutyl | Et | (Is) | 59 | 243.1391 |
| Phenyl | CH₂Cl | Et | (Vlt) | 75 | 425.1122 |
| Phenyl | 4-chlorobutyl | Et | (Iu) | 63 | 277.0983 |
| Phenyl | Benzyl | Et | (Vlv) | 73 | 467.1826 |
| Phenyl | N-phthalimidyl methyl | Et | (Vlw) | 71 | 536.1669 |
| Phenyl | 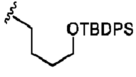 | Et | (Vlx) | 45 | 687.3103 |
| Phenyl | 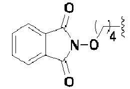 | Et | (Iy) | 76 | 404.1497 |
| Phenyl | iPr | Et | (Iz) | 76 | 229.1226 |
| Phenyl | Cyclopropyl | Et | (Iaa) | 48 | 227.1062 |
| Phenyl | Cyclobutyl | Et | (lab) | 62 | 241.1221 |
| Phenyl | Cyclohexyl | Et | (Vlac) | 75 | 459.2126 |
| Me | Me | Et | (lad) | 46 | 139.0750 |
| Et | Et | Et | (Iae) | 64 | 167.1064 |
| Et | Me | Et | (Vlaf) | 34 | 343.1511 |
| Propyl | Cyclopropyl | Et | (Vlag) | 66 | 383.1819 |
| Methyl | benzyl | Et | (Vlah) | 81 | 405.1690 |
| -CH₂CH₂Ph | Me | Et | (Vlai) | 71 | 419.1829 |
| Phenyl | Me | Me | (Iaj) | 63 | 187.0758 |
| Phenyl | Me | iPr | (Iak) | 85 | 215.1067 |
| Phenyl | Me | Phenyl | (lal) | 83 | 249.0907 |
| Et | Et | (-)-Menthyl | (Vlam) | 51 | 467.2754 |

**Table 3**

| Product number | Product name |
|---|---|
| (Ia) | 1-(ethoxyca rbo nyl)-2-phenyl-3-methylcyclopropenium hexafluorophosphate |
| (Via) | ethyl 2-methyl-3-phenyl-1-(2,4,6-trimethoxyphenyl)cycloprop-2-ene-1-carboxylate |
| (Ib) | 1-(ethoxycarbonyl)-2-(4-fluorophenyl)-3-methylcyclopropenium hexafluorophosphate |
| (Ic) | 1-(ethoxycarbonyl)-2-(4-chlorophenyl)-3-methylcyclopropenium hexafluorophosphate |
| (Id) | 1-(ethoxycarbonyl)-2-(4-bromophenyl)-3-methylcyclopropenium hexafluorophosphate |
| (le) | 1-(ethoxycarbonyl)-2-(4-iodophenyl)-3-methylcyclopropenium hexafluorophosphate |
| (If) | 1-(ethoxycarbonyl)-2-(4-acetoxyphenyl)-3-methylcyclopropenium hexafluorophosphate |
| (Ig) | 1-(ethoxycarbonyl)-2-(4-phenyphenyl)-3-methylcyclopropenium hexafluorophosphate |
| (Ih) | 1-(ethoxycarbonyl)-2-(4-trifluorophenyl)-3-methylcyclopropenium hexafluorophosphate |
| (Ii) | 1-(ethoxycarbonyl)-2-(4-(methoxycarbonyl)phenyl)-3-methylcyclopropenium hexafluorophosphate |
| (Ij) | 1-(ethoxycarbonyl)-2-(4-acrylatephenyl)-3-methylcyclopropenium hexafluorophosphate |
| (Ik) | 1-(ethoxycarbonyl)-2-(3-methylphenyl)-3-methylcyclopropenium hexafluorophosphate |
| (II) | 1-(ethoxycarbonyl)-2-(3-bromophenyl)-3-methylcyclopropenium hexafluorophosphate |
| (Im) | 1-(ethoxycarbonyl)-2-(2-chlorophenyl)-3-methylcyclopropenium hexafluorophosphate |
| (In) | 1-(ethoxycarbonyl)-2-(2-bromophenyl)-3-methylcyclopropenium hexafluorophosphate |
| (Io) | 1-(ethoxycarbonyl)-2-(1-naphthalene)-3-methylcyclopropenium hexafluorophosphate |
| (Ip) | 1-(ethoxycarbonyl)-2-(2-naphthalene)-3-methylcyclopropenium hexafluorophosphate |
| (Vlq) | ethyl 2-methyl-3-(1-tosyl-1*H*-indol-5-yl)-1-(2,4,6-trimethoxyphenyl)cycloprop-2-ene-1-carboxylate |
| (Ir) | 1-(ethoxyca rbo nyl)-2-ph enyl-3-ethylcyclopropen i u m hexafluo ro phosphate |
| (Is) | 1-(ethoxycarbonyl)-2-phenyl-3-(2-methyl)propylcyclopropenium hexafluorophosphate |
| (VIt) | ethyl 2-(chloromethyl)-3-phenyl-1-(2,4,6-trimethoxyphenyl)cycloprop-2-ene-1-carboxylate |
| (Iu) | 1-(ethoxycarbonyl)-2-phenyl-3-(4-chloro)butylcyclopropenium hexafluorophosphate |
| (VIv) | ethyl 2-benzyl-3-phenyl-1-(2,4,6-trimethoxyphenyl)cycloprop-2-ene-1-carboxylate |
| (Vlw) | ethyl 2-((1,3-dioxoisoindolin-2-yl)methyl)-3-phenyl-1-(2,4,6-trimethoxyphenyl)cycloprop-2-ene-1-carboxylate |
| (VIx) | ethyl 2-(4-((*tert*-butyldiphenylsilyl)oxy)butyl)-3-phenyl-1-(2,4,6-trimethoxyphenyl)cycloprop-2-ene-1-carboxylate |
| (Iy) | 1-(ethoxycarbonyl)-2-phenyl-3-(4-((1,3-dioxoisoindolin-2-yl)oxy)butyl)-cyclopropenium hexafluorophosphate |
| (Iz) | 1-(ethoxycarbonyl)-2-phenyl-3-isopropylcyclopropenium hexafluorophosphate |
| (Iaa) | 1-(ethoxycarbonyl)-2-phenyl-3-cyclopropylcyclopropenium hexafluorophosphate |
| (lab) | 1-(ethoxycarbonyl)-2-phenyl-3-cyclobutylcyclopropenium hexafluorophosphate |
| (Vlac) | ethyl 2-cyclohexyl-3-phenyl-1-(2,4,6-trimethoxyphenyl)cycloprop-2-ene-1-carboxylate |
| (lad) | 1-(ethoxyca rbo nyl)-2, 3-d imethylcyclopropen iu m hexafluorophosphate |
| (Iae) | 1-(ethoxyca rbonyl)-2, 3-d iethylcyclopropen iu m hexafluorophosphate |
| (Vlaf) | ethyl 2-ethyl-3-methyl-1-(2,4,6-trimethoxyphenyl)cycloprop-2-ene-1-carboxylate |
| (Vlag) | ethyl 3-propyl-2-(2,4,6-trimethoxyphenyl)-[1,1'-bi(cyclopropan)]-3-ene-2-carboxylate |
| (Vlah) | ethyl 2-benzyl-3-methyl-1-(2,4,6-trimethoxyphenyl)cycloprop-2-ene-1-carboxylate |
| (Vlai) | ethyl 2-methyl-3-phenethyl-1-(2,4,6-trimethoxyphenyl)cycloprop-2-ene-1-carboxylate |
| (Iaj) | 1-(methoxycarbonyl)-2-phenyl-3-methylcyclopropenium hexafluorophosphate |
| (Iak) | 1-(isopropoxycarbonyl)-2-phenyl-3-methylcyclopropenium hexafluorophosphate |
| (lal) | 1-(phenoxycarbonyl)-2-phenyl-3-methylcyclopropenium hexafluorophosphate |
| (Vlam) | (1*R*,2*S*,5*R*)-2-isopropyl-5-methylcyclohexyl2,3-dlethyl-1-(2,4,6-trimethoxyphenyl)cycloprop-2-ene-1-carboxylate |

The results of Table 2 and 3 show that the compounds listed in claim 10 may be prepared according to the procedure of the present Example.

### Example 3: Cyclopropenylation reaction of 1,4-dimethylbenzene (p-xylene)

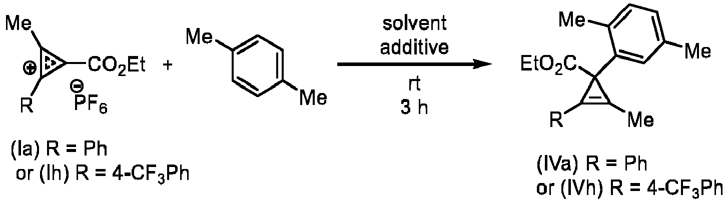

To a 10 mL oven-dried tube equipped with a stirring bar was added the corresponding compound of formula (I) as indicated in Table 4 (0.05 mmol, 18.0 mg for (Ia) and 22.0 mg for (Ih)) and the additive as indicated in Table 4 (0.075 mmol, 1.5 equiv.). The tube was sealed before being evacuated and backfilled with argon. *p*-Xylene (10.6 mg, 13 µL, 0.1 mmol) and dry solvent (1.0 mL) were added and the resulting mixture was stirred at room temperature for 3 h. Then, the reaction mixture was filtered through a pad of celite, and the solvent was removed under *vacuum.* The resulting reaction mixture was analyzed by ¹H NMR using dibromomethane as internal standard to determine the yield of the reaction.

Table 4 below shows the results obtained with different solvents and additives:

**Table 4**

| Compound of formula (I) | Solvent | Additive | ¹H NMR yield (%) |
|---|---|---|---|
| (Ia) | DCM | no | trace |
| (Ia) | HFIP | no | 20 |
| (Ia) | TFE | no | 16 |
| (Ia) | MeOH | no | trace |
| (Ia) | EtOH | no | trace |
| (Ia) | *i*PrOH | no | trace |
| (Ia) | MeCN | no | trace |
| (Ia) | HFIP | 15-C-5 | 26*^{a}* |
| (Ia) | HFIP | 4Å MS | 26*^{b}* |
| (Ia) | HFIP | NaHCO₃ | 25 |
| (Ia) | HFIP | KHCO₃ | 10 |
| (Ia) | HFIP | 2,6-Lutidine | trace |
| (Ia) | HFIP | Zn(OTf)2 | 17*^{c}* |
| (Ih) | HFIP | no | 46 |
| (Ih) | DCM | no | 38 |
| (Ih) | TFE | no | 16 |
| (Ih) | HFIP | NaHCO₃ | 50 |
| (Ih) | HFIP | Na₂CO₃ | 68 |
| (Ih) | HFIP | K2CO3 | 35 |
| (Ih) | HFIP | CsF | 92 |
| (Ih) | HFIP | NaF | 22 |
| (Ih) | HFIP | LiF | 30 |
| (Ih) | HFIP | CsBr | 13 |
| (Ih) | HFIP | CsF | 83*^{d}* |
| (Ih) | HFIP | CsF | 32*^{e}* |
| (Ih) | HFIP | CsF | 93*^{f}* |
| (Ih) | HFIP | CsF | 81*^{f,g}* |
| (Ih) | HFIP | CsF | 11*^{f,h}* |
| (Ih) | DCM | CsF | 7 |

| | | | |
|---|---|---|---|
| *^{a}* 1.0 equiv. of 15-C-5 was used. *^{b}* 30 mg 4Å MS was used. *^{c}*0.2 equiv. of Zn(OTf)₂ was used. *^{d}* 1.0 equiv. of CsF was used. *^{e}* 0.5 equiv. of CsF was used. *^{f}* 1.2 equiv. of (Ih) was used. *^{g}* 1.2 equiv. of CsF was used. *^{h}* 2.0 equiv. of CsF was used. | | | |

The results of Table 4 shows that a preferred solvent for the reaction is hexafluoroisopropanol (HFIP). The results also show that the yield of the reaction is improved when the reaction is carried out in the presence of an additive, such as a non-nucleophilic base. The results also show that caesium fluoride is a preferred base, and is preferably used in an amount of from 1 to 2 equivalents with respect to p-xylene.

### Example 4: Cyclopropenylation of different aromatic substrates

Different substrates having an aromatic or heteroaromatic moiety have been submitted to cyclopropenylation reactions using a compound of formula (I).

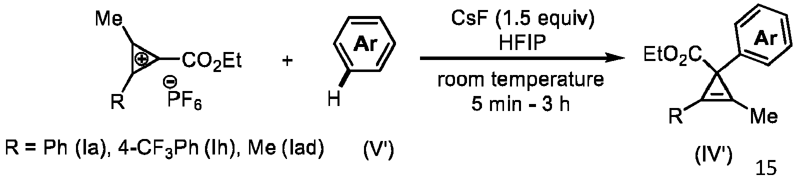

General procedure: To a 10 mL oven-dried tube equipped with a stirring bar was added the corresponding compound of formula (I) as indicated in Table 5 (0.12 mmol,) and CsF (23.0 mg, 0.15 mmol). The tube was sealed before being evacuated and backfilled with argon. A solution of the corresponding arene/heteroarene (0.1 mmol, 1.0 equiv.) in HFIP (2.0 mL) was added and the resulting mixture was stirred at room temperature. For the substrates bearing nucleophilic amine or pyridine groups, the corresponding arenes/heteroarenes of formula (V') as indicated in Table 5 (0.1 mmol, 1.0 equiv.) were treated with HBF₄ (50-55% w/w solution in Et₂O, 21 µL, 1.4 equiv.) before being added to the reaction tube. When the reaction was complete, the reaction mixture was quenched by sat. NaHCO₃. After removing the solvent, the resulting mixture was extracted with dichloromethane, dried over anhydrous sodium sulfate, analyzed by ¹H NMR (to determine the ratio of regioisomers). The crude residue was purified by column chromatography on neutral silica gel to give the corresponding cyclopropene compound of formula (IV').

Table 5 below summarizes the prepared products and obtained results.

**Table 5**

| Compound of formula (I) | Compound of formula (V') | Compound of formula (IV') | Ratio of isomers | Yield (%) |
|---|---|---|---|---|
| (Ia) | phenol | 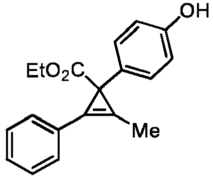 | >20:1 | 51 |
| (lad) | phenol | 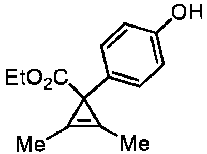 | >20:1 | 56 |
| (Ia) | anisole | 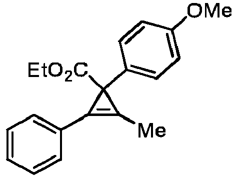 | 16:1 | 56 |
| (lad) | anisole | 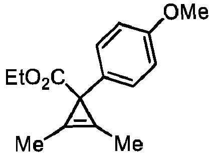 | >20:1 | 65 |
| (Ih) | *tert*-butyl methyl(phenyl) carbamate | 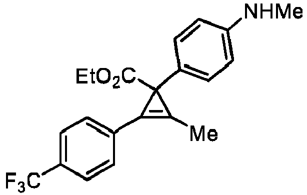 | >20:1 | 31 |
| (Ih) | 4-methyl-1,1'-biphenyl | 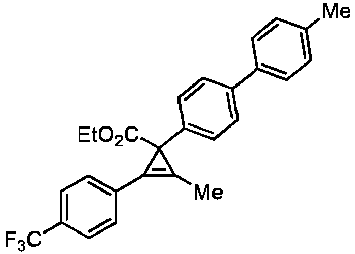 | >20:1 | 77 |
| (Ih) | toluene | 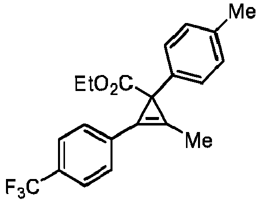 | 5.2:1 | 67 |
| (Ih) | fluorobenzene | 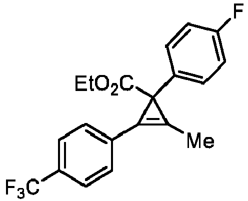 | 6:1 | 10 |
| (Ih) | benzene | 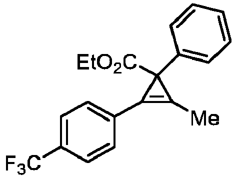 | >20:1 | 24 |
| (Ia) | p-xylene | 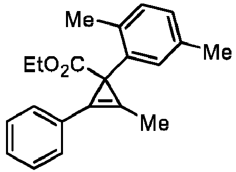 | >20:1 | 33 |
| (Ih) | p-xylene | 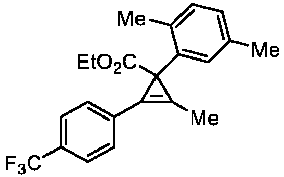 | >20:1 | 84 |
| (lad) | p-xylene | 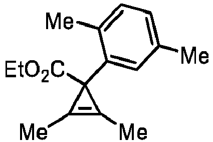 | >20:1 | 30 |
| (Ih) | m-xylene | 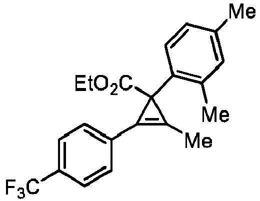 | >20:1 | 81 |
| (Ih) | o-xylene | 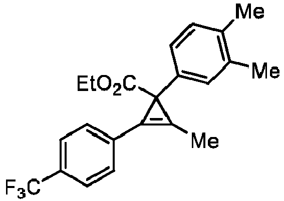 | 12:1 | 82 |
| (Ih) | 1-(*tert*-butyl)-4-methylbenzene | 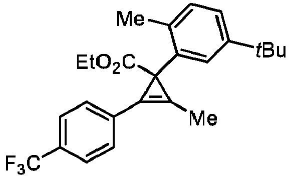 | 18:1 | 93 |
| (Ih) | 1-fluoro-3- methylbenzene | 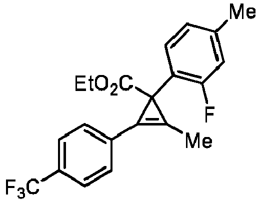 | 1.5:1 | 70 |
| (Ih) | 2-methoxybenzoa te | 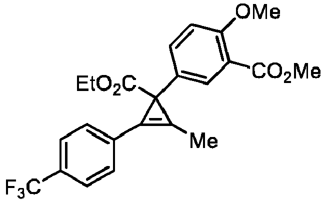 | >20:1 | 80 |
| (Ia) | 6-methoxy-1,2,3,4- tetrahydronapht halene | 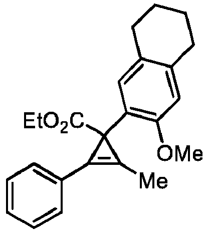 | >20:1 | 71 |
| (Ia) | mesitylene | 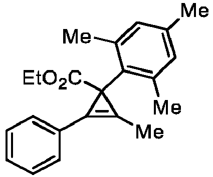 | >20:1 | 71 |
| (lad) | mesitylene | 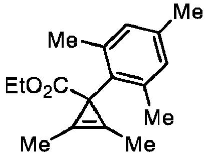 | >20:1 | 55 |
| (Ih) | naphthalene | 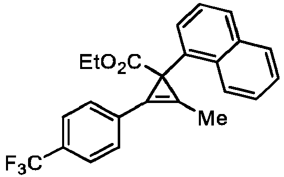 | 2.8:1 | 72 |
| (Ia) | 2-methoxynaphth alene | 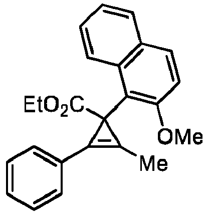 | >20:1 | 87 |
| (Ia) | 1-methoxynaphth alene | 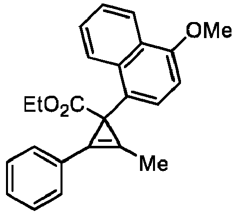 | >20:1 | 76 |
| (Ia) | 1-methyl-1H-indole | 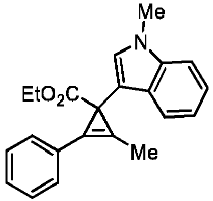 | >20:1 | 91 |
| (Ia) | methyl 1*H-*pyrrole-2- carboxylate | 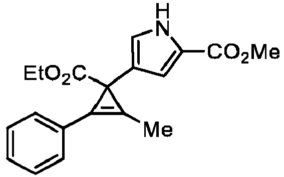 | 4.8:1 | 90 |
| (Ia) | 2-phenylthiophen e | 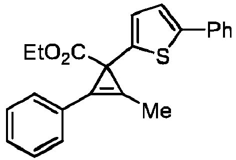 | >20:1 | 96 |
| (Ia) | 6,7-dihydrobenzofu ran-4(5*H*)-one | 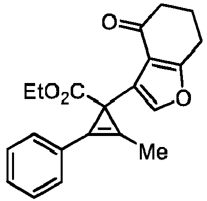 | >20:1 | 95 |
| (Ia) | 2,3-dihydrobenzofu ran | 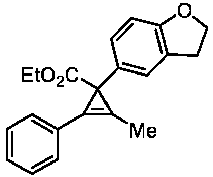 | >20:1 | 74 |
| (Ia) | 2,6-dimethoxypyridi ne | 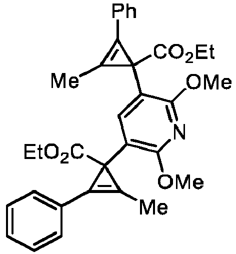 | >20:1 | 56 |
| (Ih) | indomethacin methyl ester | 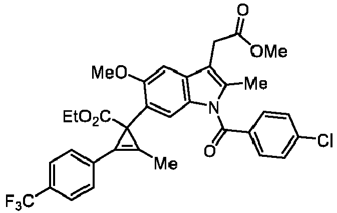 | >20:1 | 82 |
| (Ia) | estragole | 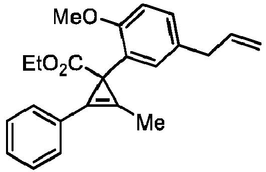 | >20:1 | 51 |
| (Ia) | carbofuran | 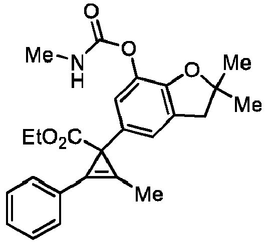 | >20:1 | 94 |
| (Ia) | chlorpropham | 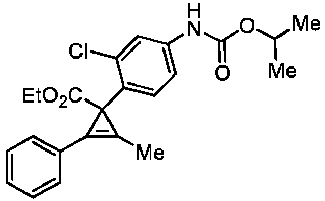 | >20:1 | 75 |
| (lad) | ciprofibrate methyl ester | 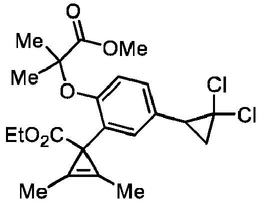 | >20:1 | 39 |
| (Ih) | 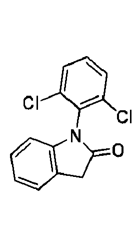 | 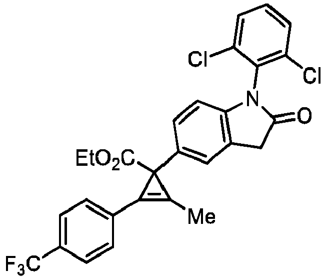 | >20:1 | 68 |
| (Ia) | desipramine hydrochloride | 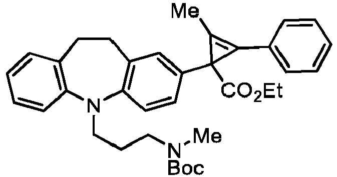 | >20:1 | 51 |
| (Ia) | desipramine hydrochloride | 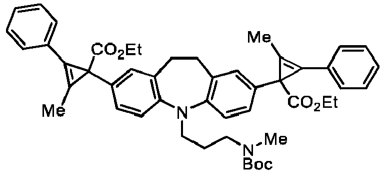 | >20:1 | 24 |
| (lad) | propanolol | 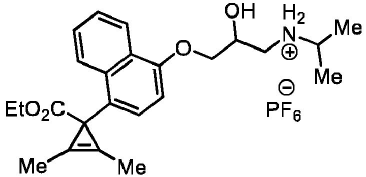 | >20:1 | 72 |
| (Ia) | gemfibrozil | 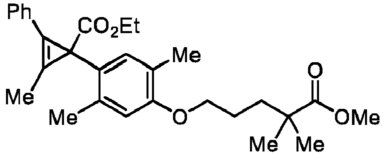 | >20:1 | 95 |
| (lad) | clopidrogrel | 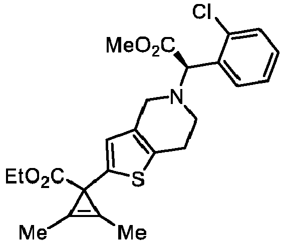 | >20:1 | 66 |
| (Ia) | aripiprazole | 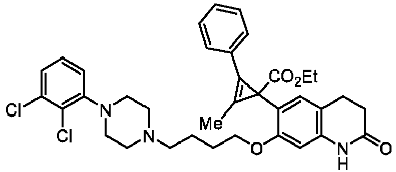 | >20:1 | 81 |
| (lad) | pyriproxyfen | 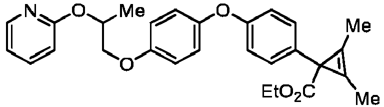 | 11:1 | 68 |
| (Ia) | estradiol dimethyl ether | 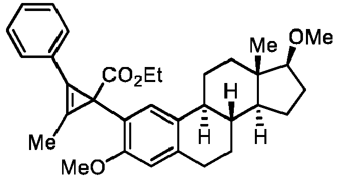 | >20:1 | 89 |
| (lad) | estradiol dimethyl ether | 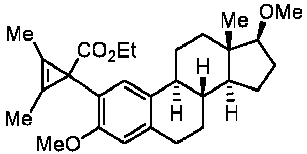 | >20:1 | 98 |
| (lad) | estrone | 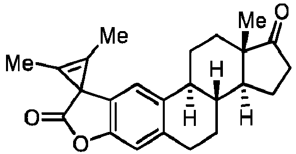 | >20:1 | 39 |
| (lad) | estrone | 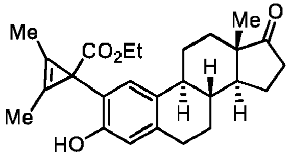 | >20:1 | 24 |
| (lad) | paroxetine hydrochloride hemihydrate | 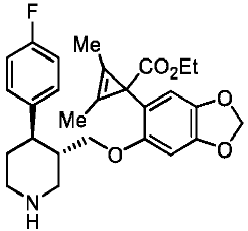 | >20:1 | 75 |
| (lad) | lopinavir | 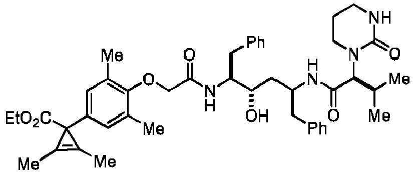 | >20:1 | 79 |
| (lad) | (S)-naproxen methyl ester | 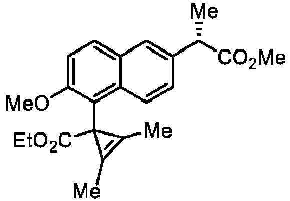 | >20:1 | 99 |
| (lad) | 5,5-difluoro-1,3,7,9-tetramethyl-5*H*-\ 4λ⁴,5λ⁴- dipyrrolo[1,2-*c*:2',1'-*f*][1,3,2]diazabo rinine | 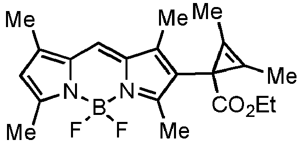 | >20:1 | 49 |
| (lad) | 5,5-difluoro-1,3,7,9,10-penta methyl-5*H*- 4λ⁴ 5λ⁴-dipyrrolo[1,2- *c*:2',1'-*f*][1,3,2]diazabo rinine | 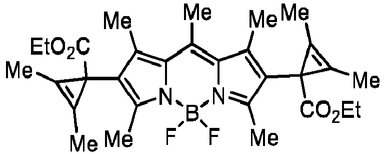 | >20:1 | 52 |

The results of Table 5 show that the cyclopropenylation process of the invention advantageously takes place with high regioselectivity and with reaction substrates bearing different functional groups. The process of the invention is thus advantageously highly versatile because it may be carried out on a broad range of substrates, including substrates bearing functional groups. As a further advantage, the resulting cyclopropenylated compound may be readily converted into further compounds using the high reactivity of the formed cyclopropenyl ring. Such additional reactions include, for instance, catalytic hydrogenation, cycloaddition reaction, rhodium-catalyzed hydrothiolation, decarboxylation, hydrostannation, hydroborylation, synthesis of highly functionalized cyclopropenium cations, intermolecular Pauson-Khand reaction, and cycloisomerization.

### Example 5: Cyclopropenylation of organometallic compounds

General procedure: To a 10 mL oven-dried reaction tube was added the compound of formula (Ia) (0.1 mmol, 35 mg). The tube was sealed before being evacuated and backfilled with argon. Dry, degassed dichloromethane (1.0 mL) was added, and the solution was cooled to 0 °C. A solution of the corresponding organometallic reagent as indicated in Table 6 (0.15 mmol, 1.5 eq.) was added dropwise and the reaction was stirred for 15 minutes. The reaction mixture was then quenched with sat. NH₄Cl, extracted three times with dichloromethane, dried on anhydrous sodium sulfate and concentrated under reduced pressure. After analysis of the crude by ¹H NMR, the crude residue was purified either by filtration on a neutral silica gel plug, or by column chromatography on neutral silica gel to give the corresponding cyclopropene compound.

Table 6 below summarizes the prepared products and obtained results.

**Table 6**

| Organometallic reagent | Product | Ratio of isomers | Yield (%) |
|---|---|---|---|
| phenyl magnesium chloride | 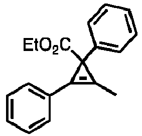 | >20:1 | 95 |
| methyl magnesium bromide | 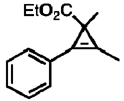 | >20:1 | 77 |
| vinyl magnesium bromide | 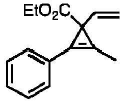 | >20:1 | 88 |
| 4-fluorophenylmagnesium bromide | 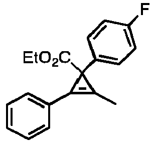 | >20:1 | 73 |
| 4-methoxyphenylmagnesium bromide | 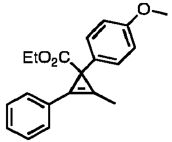 | >20:1 | 63 |
| p-tolylmagnesium bromide | 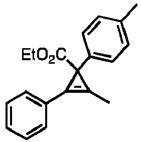 | >20:1 | 75 |
| 4-chlorophenylmagnesium bromide | 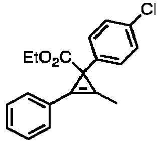 | >20:1 | 77 |
| 3,5-bis(trifluoromethyl)phenylmagnesium bromide | 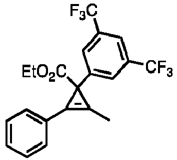 | >20:1 | 70 |
| 3-methoxyphenylmagnesium bromide | 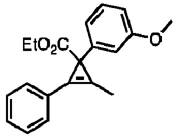 | >20:1 | 50 |
| benzylmagnesium bromide | 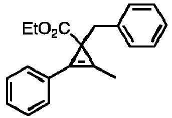 | >20:1 | 75 |
| diethylzinc | 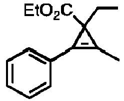 | >20:1 | 73 |
| naphthalen-1-ylboronic acid | 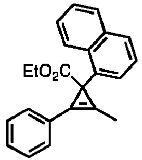 | >20:1 | 76 |
| p-tolylboronic acid | 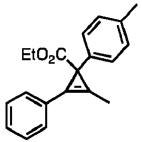 | >20:1 | 60 |
| p-tolylboronic acid pinacol ester | 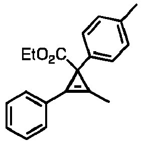 | >20:1 | 58 |

The results of Table 6 show that the compounds of formula (I) according to the invention are suitable as cyclopropenylation reagents in reactions with organometallic compounds such as Grignard reagents, organozinc compounds and organoboron compounds.

### Example 6: Further modification of cyclopropenylated compounds

In Examples 6a-6e below, the starting materials were prepared following the procedures of Example 4. This Example shows that the cyclopropenyl motif is synthetically versatile and may be used in a further step for structurally modifying the prepared compound.

### Example 6a: Pd-catalyzed hydrogenation

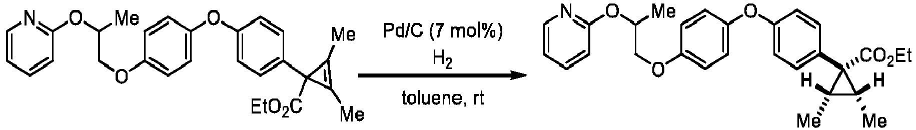

7% Pd/C (7.4 mg, 0.007 mmol) was added to a solution of cyclopropene derivative (46.0 mg, 0.10 mmol) in toluene (3.0 mL). After being purged with hydrogen, the suspension was stirred vigorously under hydrogen atmosphere overnight. The reaction mixture was then filtered through a short plug of celite and concentrated in vacuo. The residue was purified with column chromatography (hexane/ethyl acetate = 6/1) to give the desired cyclopropane compound as a colourless oil (40.5 mg, 88% yield).

### Example 6-b: Rh(I)-catalyzed cycloisomerization

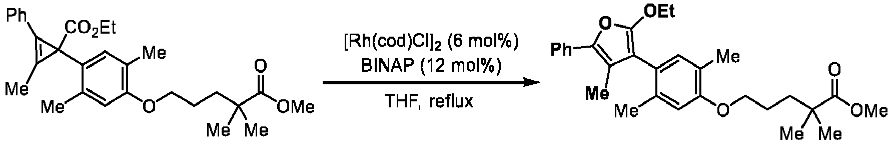

To a 10 mL oven-dried tube equipped with a stirring bar was added [Rh(cod)Cl]₂ (1.5 mg, 6 mol%) and (rac)-BINAP (3.8 mg, 12 mol%). After being evacuated and backfilled with argon, 0.5 mL of dry THF was added and the mixture was stirred at room temperature for 20 minutes. Then a solution of the corresponding cyclopropene derivative (23.0 mg, 0.05 mmol) in THF (1.0 mL) was added and the resulting mixture was heated at 70 °C. When the reaction was complete (monitored by TLC), the reaction mixture was passed through a short pad of celite and washed with dichloromethane. The solvent was removed. Purification by flash chromatography on neutral silica gel (hexane/ethyl acetate = 8/1 to 4/1) provided the corresponding furan derivative as a yellow oil (19.1 mg, 82% yield).

### Example 6c: Intermolecular Pauson-Khand reaction

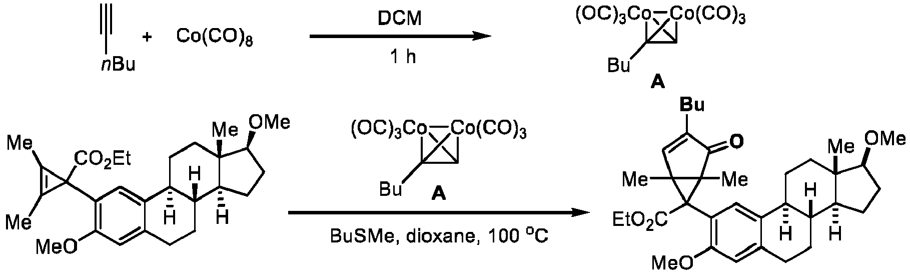

Compound A was synthesized according to the method disclosed in Org. Lett. 2011, 3, 3193-3196 (incorporated herein by reference). To a 25 mL Schlenk tube containing the cyclopropenylated compound (39.0 mg, 0.09 mmol) was added A (110.0 mg, 0.30 mmol), n-butyl methyl sulfide (416 mg, 4.00 mmol), and 1,4-dioxane (3.0 mL). The reaction was carried out at 100 °C for 2 h, and chromatographed over silica gel (hexane/ethyl acetate = 6/1) to give the desired compound as a white solid (38.5 mg, 79% yield, 1.2:1 mixture of diastereoisomers as determined by ¹H NMR.

### Example 6d: Au(I)-catalyzed cycloisomerization of cyclopropene compound

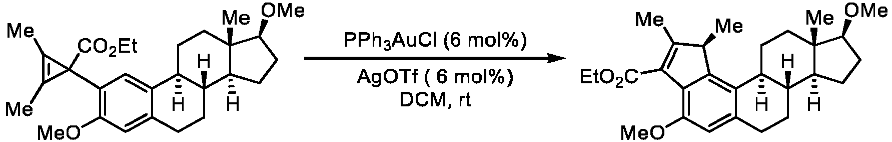

To a 10 mL oven-dried tube equipped with a stirring bar was added AuClPPh₃ (1.6 mg, 6 mol%) and AgOTf (0.8 mg, 6 mol%). After being evacuated and backfilled with argon, 0.5 mL of dry dichloromethane was added and the mixture was stirred at room temperature for 5 minutes. Then a solution of the corresponding cyclopropene compound (22.0 mg, 0.05 mmol) in dichloromethane (1.0 mL) was added. The solution turned yellow immediately. The reaction was monitored with TLC. After the substrate was consumed, the solvent was removed in vacuo and the crude product was purified by preparative TLC (hexane/diethyl ether = 3/2) to give the corresponding indene compound as a yellow oil (9.4 mg, 43% yield).

### Example 6e: Synthesis of cyclopropenium cations

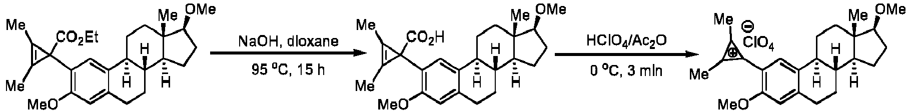

### Example 6e-1

Aqueous NaOH (34 mL of 1N solution) was added to a 200 mL flask containing the reaction product of (lad) with estradiol dimethyl ether prepared according to Example 4 (1.3 g, 3.0 mmol), 150 mL dioxane and 21 mL water. The mixture was stirred at 95 °C until full conversion of the starting material. Dioxane was removed in vacuo and the residue was acidified to pH 1-3 with 2N HCI solution. The resulting mixture was extracted with dichloromethane (20 mL x 4) and dried over Na₂SO₄. After removing the solvent, the crude residue was purified by column chromatography on silica gel (hexane/ethyl acetate = 2/1 to 1/1) to afford the corresponding acid derivative as a white foam (1.2 g, 95% yield).

4.0 g of freshly prepared HClO₄ solution in acetic anhydride (0.2 g of 70% HClO₄ in 5.6 g acetic anhydride) was added slowly to a reaction flask containing the acid compound as prepared above (410 mg, 1.0 mmol) at 0 °C. The mixture turned red immediately and gas evolution was observed. After 3 minutes, diethyl ether (3 mL) and hexane (3 mL) were added, and the mixture was stirred vigorously for additional 1 minute. Two layers appeared after standing still for 1 minute, and the upper layer was removed carefully. Then the residue was washed with diethyl ether (3 mL x 4), dried under vacuum to give the cyclopropenium compound as a red foam (380 mg, 82% yield).

### Example 6e-2

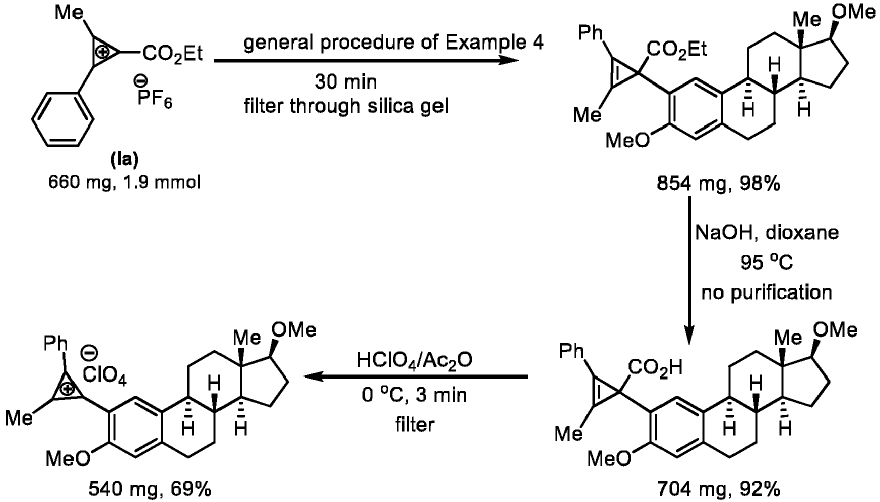

Aqueous NaOH (19 mL of 1N solution) was added to a 200 mL flask containing a cyclopropenylated compound as prepared following the general procedure of Example 4 from the compound of formula (Ia) and estradiol dimethyl ether (810 mg, 1.6 mmol), 81 mL dioxane and 10 mL water. The mixture was stirred at 95 °C until full conversion of the starting material. Dioxane was removed in vacuo and the residue was acidified to pH 1-3 with 2N HCI solution. The resulting mixture was extracted with dichloromethane (20 mL x 4) and dried over Na₂SO₄. After removing the solvent, the corresponding acid was obtained as a white foam (704 mg, 92% yield).

6.0 g of freshly prepared HClO₄ solution in acetic anhydride (0.25 g of 70% HClO₄ in 7.0 g acetic anhydride) was added slowly to a reaction flask containing the previously made acid (704 mg, 1.5 mmol) at 0 °C. The mixture turned yellow immediately and gas evolution was observed. After 3 minutes, diethyl ether (4 mL) was added, and the mixture was stirred vigorously for an additional minute. The product was collected by filtration, washed with diethyl ether (4 mL x 4), dried under high vacuum to give the cyclopropenium compound as a yellow solid (540 mg, 69% yield).

### Example 6e-3: addition of Grignard reagent to cyclopropenium salt

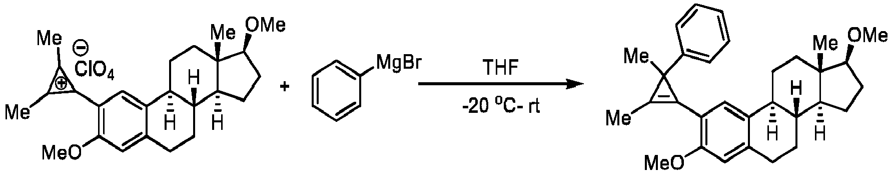

To a 25 mL oven-dried flask equipped with a stirring bar was added the cyclopropenium starting material (78.5 mg, 0.17 mmol). After being evacuated and backfilled with argon, 3.0 mL of dry THF was added and the reaction was cooled to -20 °C. Then phenylmagnesium bromide (0.34 mL, 1M in THF) was added slowly. The mixture turned from red to yellow in 20 minutes. The reaction mixture was passed through a short pad of celite and washed with dichloromethane. The solvent was removed under vacuum. Purification by flash chromatography on neutral silica gel (hexane/ethyl acetate = 25/1 to 15/1) provided the corresponding cyclopropene derivative as a white foam (46.1 mg, 61% yield, 1:1 mixture of diastereoisomers as determined by ¹H NMR).

### Example 6e-4: synthesis of indene and/or naphthol from the product of Example 6e-3

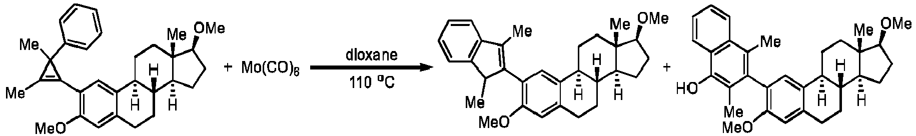

A screw capped reaction tube equipped with a magnetic bar was charged with cyclopropene compound as prepared in Example 6e-3 (21.0 mg, 0.05 mmol, 1.0 equiv.), MO(CO)₆ (64.6 mg, 0.06 mmol, 1.2 equiv.) and flushed with argon. Subsequently, 1,4-dioxane (1.0 mL) was added. The tube was sealed with a Teflon-lined screw cap, and stirred vigorously at 110 °C for 2 h. After cooling to room temperature, the solvent was concentrated in vacuo. Purification of the resulting crude residue by column chromatography on silica gel (hexane/ethyl acetate = 4/1 to 2/1) provided the corresponding indene product (colourless oil, 15.4 mg, 70% yield, 1:1 mixture of diastereoisomers as determined by ¹H NMR) and naphthol product (colourless oil, 5.9 mg, 26% yield, 1:1 mixture of diastereoisomers as determined by ¹H NMR).

### Example 6e-5: addition of hydride to cyclopropenium salt of Example 6e-1 and Example 6e-2

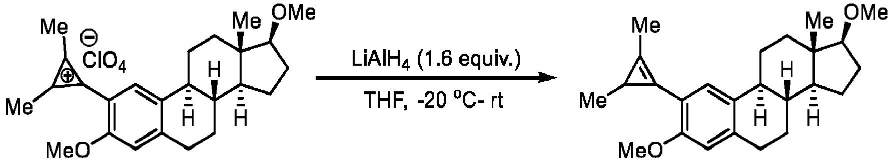

To a 25 mL oven-dried flask equipped with a stirring bar was added the cyclopropenium salt of Example 6e-1 (400.0 mg, 0.86 mmol). After being evacuated and backfilled with argon, 9.0 mL of dry THF was added and the reaction was cooled to -20 °C. Then lithium aluminium hydride (52.0 mg, 1.40 mmol) was added in one portion. The mixture turned from red to yellow in 20 minutes. The reaction mixture was passed through a short pad of celite and washed with dichloromethane. The solvent was removed and the resulting reaction mixture was analyzed by ¹H NMR. Purification by flash chromatography on neutral silica gel (hexane/ethyl acetate = 25/1 to 15/1) provided the corresponding cyclopropene compound as a white foam (221.0 mg, 70% yield, 1:1 mixture of diastereoisomers as determined by ¹H NMR).

The same procedure was carried out with the product of Example 6e-2, affording a yield of the corresponding cyclopropenylated compound of 80% (1:1 mixture of diastereoisomers).

### Example 6e-6: addition of vinyl magnesium bromide to cyclopropenium salt of Example 6e-1

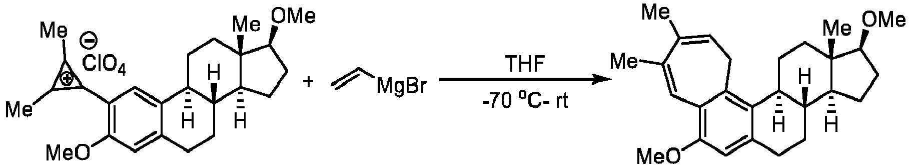

To a 25 mL oven-dried flask equipped with a stirring bar was added the cyclopropenium salt of Example 6e-1 (46.4 mg, 0.10 mmol). After being evacuated and backfilled with argon, 2.0 mL of dry THF was added and the reaction was cooled to -70 °C. Then vinylmagnesium bromide (0.17 mL, 1M in THF) was added slowly. The mixture turned from red to yellow in 20 minutes. Then neutral silica gel was added to the reaction mixture. The solvent was removed and the crude residue was purified by column chromatography on neutral silica gel (hexane/ethyl acetate = 25/1 to 15/1) to give the corresponding reaction product as a colourless oil (18.0 mg, 46% yield).

### Example 6e-7: 1,3-dipolar cycloaddition reactions

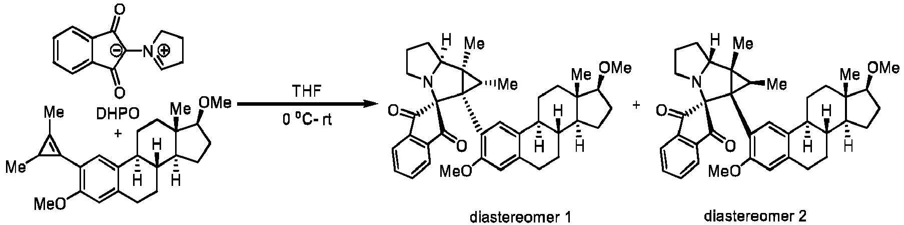

To a 10 mL oven-dried tube equipped with a stirring bar was added the cyclopropene compound as prepared in Example 6e-5 (15.0 mg, 0.04 mmol) and DHPO (13.0 mg, 0.06 mmol). After being evacuated and backfilled with argon, 1.0 mL of dry THF was added at 0 °C. The mixture was warmed to room temperature slowly. When the reaction was complete (monitored with TLC), the solvent was removed in vacuo. The residue was analyzed by ¹H NMR as a 1:1 mixture of diastereoisomers. The crude product was purified by preparative TLC (dichloromethane/ethyl acetate = 7/1) to give the corresponding compound (16.5 mg, 71% yield).

### Example 6e-8: Diels-Alder reaction

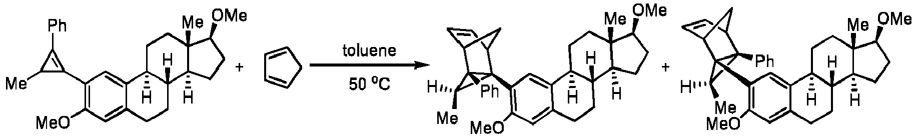

A solution of cyclopropene compound from Example 6e-2 (8.0 mg, 0.02 mmol) and cyclopentadiene (22.0 mg, 0.30 mmol) in toluene (1 mL) was stirred at 50 °C for 12 h. The residue after rotary evaporation was purified by column chromatography (hexane/ethyl acetate = 16/1 to 10/1) to afford the desired cycloadduct as a yellow oil (7.1 mg, 66% yield, 1:1 mixture of diastereoisomers as determined by ¹H NMR).

### Example 6e-9: Hydroborylation of the product of Example 6e-5

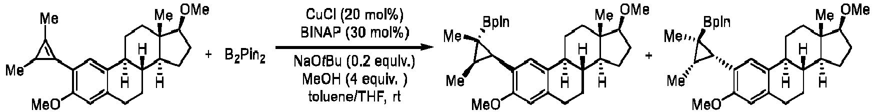

A dried Schlenk flask was charged with CuCI (1.0 mg, 20 mol%), (rac)-BINAP (9.3 mg, 30 mol%), B₂Pin₂ (26.0 mg, 0.10 mmol, 2.0 equiv.), NaOtBu (1.2 mg, 25 mol%) and anhydrous toluene (1.0 mL) under argon atmosphere. After the mixture was stirred at room temperature for 40 minutes, a solution of the product of Example 6e-5 (18.0 mg, 0.05 mmol) in anhydrous THF (1.0 mL) was added, followed by anhydrous methanol (8.0 µL, 4.0 equiv.). The resulting mixture was stirred at room temperature until full consumption of the starting material (monitored with TLC), then filtered through celite, concentrated in vacuo, analyzed by ¹H NMR. The residue was purified by column chromatography (hexane/ethyl acetate = 15/1 to 9/1) to afford the desired product as a yellow oil (14.1 mg, 57% yield, 1:1 mixture of diastereoisomers as determined by ¹H NMR).

### Example 6e-10: Hydrostannation of the product of Example 6e-5

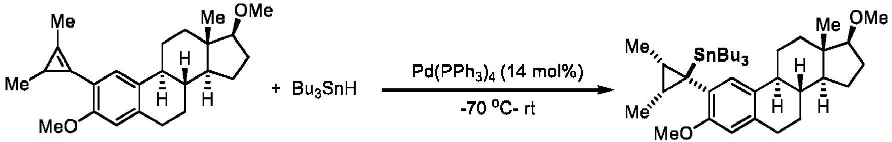

To a 10 mL oven-dried tube equipped with a stirring bar was added Pd(PPh₃)₄ (4.0 mg, 14 mol %). After being evacuated and backfilled with argon, 0.5 mL of dry THF was added. The resulting mixture was stirred for 10 minutes. The solution was cooled to -70 °C, and tributyltin hydride (10 µL, 0.04 mmol) was added. The reaction mixture was stirred at -70 °C for 1 minute, after which the product of Example 6e-5 (9.0 mg, 0.025 mmol) in THF (0.5 mL) was added, and the mixture was warmed to -30 °C slowly. When the reaction was complete (monitored with TLC), the solvent was removed in vacuo and the resulting reaction mixture was analyzed by ¹H NMR. The crude product was purified by column chromatography on silica gel (hexane/ethyl acetate = 16/1 to 10/1) to yield the corresponding cyclopropane compound as a colourless oil (7.1 mg, 44% yield).

### Example 6e-11: Hydrothiolation of the product of Example 6e-5

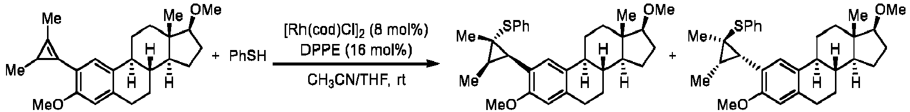

To a 10 mL oven-dried tube equipped with a stirring bar was added [Rh(cod)Cl]₂ (2.0 mg, 8 mol%), DPPE (3.3 mg, 16 mol%). After being evacuated and backfilled with argon, 1.0 mL of dry MeCN was added. The resulting mixture was stirred for 10 minutes. Thiophenol (11.0 mg, 0.10 mmol) was added followed by the product of Example 6e-5 (18.0 mg, 0.05 mmol) in THF (1.0 mL). When the reaction was complete (monitored with TLC), the solvent was removed in vacuo and the resulting reaction mixture was analyzed by ¹H NMR. The crude product was purified by preparative TLC (hexane/acetonitrile = 7/1) to give the corresponding product as a yellow oil (14.4 mg, 61% yield, 1:1 mixture of diastereoisomers as determined by ¹H NMR).

### Example 6e-12

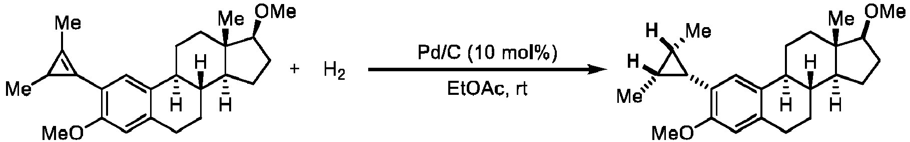

10% Pd/C (7.4 mg, 0.007 mmol) was added to a solution of cyclopropene derivative produced in Example 6e-5 (27.0 mg, 0.07 mmol) in ethyl acetate (2.0 mL). After being purged with hydrogen, the suspension was stirred vigorously under hydrogen atmosphere overnight. The reaction mixture was then filtered through a short plug of celite, and concentrated in vacuo. The residue was analyzed by ¹H NMR as a 16:3:1 mixture of diastereoisomers. Purification by flash chromatography on silica gel (hexane/ethyl acetate = 6/1) provided the desired cyclopropane compound as a colourless oil (22.1 mg, 81% yield).

## Claims

1. A compound of formula (I) 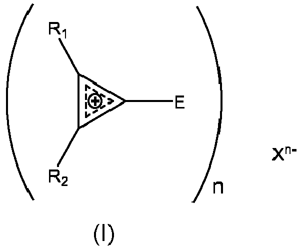
wherein E is an electron withdrawing group selected from the group consisting of (C₁-C₆)haloalkyl, a radical of formula -COOR₃, a formyl group (-CHO), (C₁-C₆)alkylcarbonyl, carboxyl (-COOH), nitro, (C₁-C₆)alkyloxysulfonyl, a group of formula -P(O)(O(C₁-C₆)alkyl)₂, nitrile and an aromatic ring system comprising from 1 to 2 6-membered aromatic rings, the members of the rings being selected from the group consisting of C, CH and N, being at least one member N, and the rings being further optionally substituted at any available position with one or more group selected from halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, nitrile, a formyl group and nitro;
each of R₁, R₂ and R₃ is independently selected from the group consisting of (C₁-C₁₂)alkyl, (C₂-C₁₂)alkenyl and a ring system RS comprising from 1 to 5 rings, each ring being saturated, unsaturated or aromatic, each ring comprising from 3 to 7 members selected from the group consisting of C, CH, CH₂, O, N, NH, NR₄ and S, each ring being isolated, bridged or fused; wherein each of the (C₁-C₁₂)alkyl, (C₂-C₁₂)alkenyl and the ring system RS is optionally substituted at any available position with one or more radicals selected from the group consisting of halogen, (C₆-C₂₀)aryl, (C₅-C₂₀)heteroaryl, (C₃-C₁₂)cycloalkyl, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a formyl group, a nitro group, a radical of formula -PG₁, a radical of formula -OPG₁ and a radical of formula -NR₄R₅;
wherein PG₁ is selected from the group consisting of *N*-phthalimidyl and a radical of formula -SiR₆R₇R₈;
wherein R₄ is selected from the group consisting of (C₁-C₆)alkyloxycarbonyl, benzyloxycarbonyl, 9-fluorenylmethoxycarbonyl; (C₁-C₆)alkylsulfonyl and phenylsulfonyl wherein the phenyl ring is optionally substituted with one or more radicals selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, nitrile, a formyl group and nitro;
R₅ is hydrogen or (C₁-C₆)alkyl;
each of R₆, R₇ and R₈ is independently selected from the group consisting of (C₁-C₆)alkyl and phenyl;
Xⁿ⁻ is an anion selected from the group consisting of hexafluorophosphate, hexafluoroantimonate, hexafluoroaluminate, hexafluorogermanate, hexafluorozirconate, hexafluoroarsenate, hexafluorotitanate, hexachloroantimonate, hexafluorosilicate, tetrafluoroberyllate, hexachlorophosphate, hexachlorosilicate, perchlorate, trifluoromethanesulfonate, tetrafluoroborate and B(Ar)₄⁻ wherein Ar is a phenyl ring optionally substituted at any available position with a group selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, nitrile, a formyl group and nitro;
and n is an integer of from 1 to 3.

2. Compound according to claim 1 wherein E is a group of formula -COOR₃.

3. Compound according to any one of claims 1 to 2 wherein R₁ is a (C₁-C₁₂)alkyl or a (C₃-C₈)cycloalkyl group, said (C₁-C₁₂)alkyl and (C₃-C₈)cycloalkyl groups being optionally substituted at any available position with one or more radicals selected from the group consisting of halogen, (C₆-C₂₀)aryl, (C₅-C₂₀)heteroaryl, (C₃-C₁₂)cycloalkyl, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, (C₂-C₆)alkenylcarbonyloxy, (C₂-C₆)alkenyloxycarbonyl, nitrile, a formyl group, a nitro group, a radical of formula -PG₁, a radical of formula -OPG₁ and a radical of formula -NR₄R₅.

4. Compound according to any one of claims 1 to 3 wherein R₁ is selected from the group consisting of methyl, ethyl, n-propyl, *iso*-propyl, iso-butyl, chloromethyl, 4-chloro-n-butyl, benzyl, *N*-phthalimidylmethyl, 4-((tert-butyldiphenylsilyl)oxy)butyl, 4-((N-phthalimidyl)oxy)butyl, cyclopropyl, cyclobutyl, cyclohexyl and 2-phenylethyl.

5. Compound according to any one of claims 1 to 4 wherein R₂ is selected from the group consisting of (C₁-C₁₂)alkyl and a ring system comprising from 1 to 2 aromatic rings, each ring comprising from 5 to 6 members selected from the group consisting of C, CH, O, N, NH, NR₄ and S, each ring being isolated or fused; said rings being further optionally substituted at any available position with one or more radicals selected from the group consisting of halogen, (C₁-C₆)alkyl, (C₁-C₆)haloalkyl, (C₁-C₆)alkyloxy, (C₁-C₆)alkylcarbonyl, (C₁-C₆)alkylcarbonyloxy, (C₁-C₆)alkyloxycarbonyl, (C₂-C₆)alkenylcarbonyloxy and (C₂-C₆)alkenyloxycarbonyl.

6. Compound according to any one of claims 1 to 5 wherein R₂ is selected from the group consisting of methyl, 1-naphthyl, 2-naphthyl, *N*-(p-toluenesulfonyl)indol-5-yl, 4-phenylphenyl and phenyl optionally substituted with one or more radicals selected from the group consisting of halogen, trifluoromethyl, acetoxy, methyloxycarbonyl, vinylcarbonyloxy, methyl, ethyl and propyl.

7. Compound according to any one of claims 1 to 6 wherein R₃ is selected from the group consisting of (C₁-C₁₂)alkyl and a ring system comprising from 1 to 2 rings, each ring being saturated, unsaturated or aromatic, each ring comprising from 3 to 7 members selected from the group consisting of C, CH, CH₂, O, N, NH, NR₄ and S, each ring being isolated, bridged or fused; wherein said ring system is optionally substituted at any available position with a (C₁-C₆)alkyl group.

8. Compound according to any one of claims 1 to 7 wherein R₃ is selected from the group consisting of methyl, ethyl, *iso*-propyl, phenyl and 2-*iso*-propyl-5-methyl-cyclohex-1-yl.

9. Compound according to any one of claims 1 to 8 wherein Xⁿ⁻ is hexafluorophosphate.

10. Compound according to any one of claims 1 to 9 that is selected from the group consisting of:
1-(ethoxycarbonyl)-2-phenyl-3-methylcyclopropenium hexafluorophosphate;
1-(ethoxycarbonyl)-2-(4-fluorophenyl)-3-methylcyclopropenium hexafluorophosphate;
1-(ethoxycarbonyl)-2-(4-chlorophenyl)-3-methylcyclopropenium hexafluorophosphate;
1-(ethoxycarbonyl)-2-(4-bromophenyl)-3-methylcyclopropenium hexafluorophosphate;
1-(ethoxycarbonyl)-2-(4-iodophenyl)-3-methylcyclopropenium hexafluorophosphate;
1-(ethoxycarbonyl)-2-(4-acetoxyphenyl)-3-methylcyclopropenium hexafluorophosphate;
1-(ethoxycarbonyl)-2-(4-phenyphenyl)-3-methylcyclopropenium hexafluorophosphate;
1-(ethoxycarbonyl)-2-(4-trifluorophenyl)-3-methylcyclopropenium hexafluorophosphate;
1-(ethoxycarbonyl)-2-(4-(methoxycarbonyl)phenyl)-3-methylcyclopropenium hexafluorophosphate;
1-(ethoxycarbonyl)-2-(4-acrylatephenyl)-3-methylcyclopropenium hexafluorophosphate;
1-(ethoxycarbonyl)-2-(3-methylphenyl)-3-methylcyclopropenium hexafluorophosphate;
1-(ethoxycarbonyl)-2-(3-bromophenyl)-3-methylcyclopropenium hexafluorophosphate;
1-(ethoxycarbonyl)-2-(2-chlorophenyl)-3-methylcyclopropenium hexafluorophosphate;
1-(ethoxycarbonyl)-2-(2-bromophenyl)-3-methylcyclopropenium hexafluorophosphate;
1-(ethoxycarbonyl)-2-(1-naphthalene)-3-methylcyclopropenium hexafluorophosphate;
1-(ethoxycarbonyl)-2-(2-naphthalene)-3-methylcyclopropenium hexafluorophosphate;
1-(ethoxycarbonyl)-2-(methyl)-3-(1-tosyl-1*H*-indol-5-yl)cyclopropenium hexafluorophosphate;
1-(ethoxycarbonyl)-2-phenyl-3-ethylcyclopropenium hexafluorophosphate;
1-(ethoxycarbonyl)-2-phenyl-3-(2-methyl)propylcyclopropenium hexafluorophosphate;
1-(ethoxycarbonyl)-2-phenyl-3-(chloromethyl)cyclopropenium hexafluorophosphate;
1-(ethoxycarbonyl)-2-phenyl-3-(4-chloro)butylcyclopropenium hexafluorophosphate;
1-(ethoxycarbonyl)-2-phenyl-3-benzylcyclopropenium hexafluorophosphate;
1-(ethoxycarbonyl)-2-phenyl-3-((*N*-phthalimidyl)methyl)cyclopropenium hexafluorophosphate;
1-(ethoxycarbonyl)-2-(4-((tert-butyldiphenylsilyl)oxy)butyl)-3-phenylcyclopropenium hexafluorophosphate;
1-(ethoxycarbonyl)-2-phenyl-3-(4-((1,3-dioxoisoindolin-2-yl)oxy)butyl)cyclopropenium hexafluorophosphate;
1-(ethoxycarbonyl)-2-phenyl-3-isopropylcyclopropenium hexafluorophosphate;
1-(ethoxycarbonyl)-2-phenyl-3-cyclopropylcyclopropenium hexafluorophosphate;
1-(ethoxycarbonyl)-2-phenyl-3-cyclobutylcyclopropenium hexafluorophosphate;
1-(ethoxycarbonyl)-2-phenyl-3-cyclohexylcyclopropenium hexafluorophosphate;
1-(ethoxycarbonyl)-2,3-dimethylcyclopropenium hexafluorophosphate;
1-(ethoxycarbonyl)-2,3-diethylcyclopropenium hexafluorophosphate;
1-(ethoxycarbonyl)-2-ethyl-3-methylcyclopropenium hexafluorophosphate;
1-(ethoxycarbonyl)-2-propyl-3-cyclopropylcyclopropenium hexafluorophosphate;
1-(ethoxycarbonyl)-2-benzyl-3-methylcyclopropenium hexafluorophosphate;
1-(ethoxycarbonyl)-2-(2-phenyl)ethyl-3-methylcyclopropenium hexafluorophosphate;
1-(methoxycarbonyl)-2-phenyl-3-methylcyclopropenium hexafluorophosphate;
1-(isopropoxycarbonyl)-2-phenyl-3-methylcyclopropenium hexafluorophosphate;
1-(phenoxycarbonyl)-2-phenyl-3-methylcyclopropenium hexafluorophosphate and
1-(2-isopropyl-5-methylcyclohexyloxycarbonyl)-2,3-diethylcyclopropenium hexafluorophosphate.

11. Process for the preparation of a compound of formula (I) as defined in any one of claims 1 to 10, comprising the step of contacting a compound of formula (II) with a compound of formula (III) 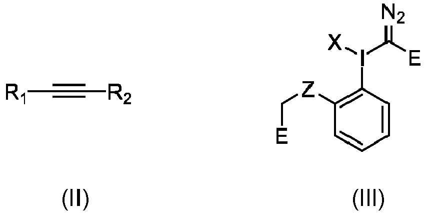
in the presence of a catalytically effective amount of a rhodium(II) catalyst,
wherein X, R₁, R₂ and E are as defined in any one of claims 1 to 10; and wherein,
in the compound of formula (III), X is attached to the iodine atom via a covalent or ionic bond and Z is a divalent group selected from the group consisting of -COO-, -C((C₁-C₆)alkyl)₂O-, -SO₂-O-, -NR-O-, -B(OR)-O, -S-O-, and -P(O)(OR)-O-, wherein R is H or (C₁-C₆)alkyl.

12. Process according to claim 11 wherein at least one of the conditions (i) to (vi) is satisfied:
(i) the rhodium(II) catalyst is bis[rhodium(α,α,α',α'-tetramethyl-1,3-benzenedipropionic acid)];
(ii) the catalyst is present in an amount of 1 mole of catalyst per each 100 moles of compound of formula (III);
(iii) the molar ratio of the compound of formula (II) to the compound of formula (III) is of from 1:1 to 3:1;
(iv) the process is carried out at a temperature of below - 40 °C;
(v) the process is carried out in a chlorinated organic solvent; and
(vi) the compound of formula (I) is isolated by precipitation from a (C₄-C₇)alkane solvent.

13. Use of a compound of formula (I) as defined in any one of claims 1 to 10 as a cyclopropenylation reagent.

14. Process for the preparation of a compound comprising a moiety of formula (IV) 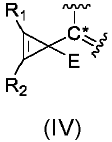 said process comprising the step of reacting a compound of formula (I) as defined in any one of claims 1 to 10 with a compound comprising a moiety of formula (V) 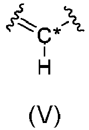 wherein C* represents a carbon atom comprised in an aromatic or heteroaromatic ring system and wherein said process converts the moiety of formula (V) in the moiety of formula (IV).

15. Process according to claim 14 wherein at least one of the conditions (a) to (c) is satisfied:
the process is carried out in a solvent selected from the group consisting of dichloromethane, hexafluoroisopropanol and trifluoroethanol; preferably, the solvent is hexafluoroisopropanol;
the process is carried out in the presence of an additive selected from the group consisting of alkaline salts of hydrogen carbonate, alkaline salts of fluoride, alkaline salts of carbonate, crown ether 15-C-5 and molecular sieves; preferably, the additive is caesium fluoride; and
when the process is carried out in the presence of an additive as defined in (ii), said additive is present in an amount of 1.5 mole per each mole of the compound comprising the moiety of formula (V).

16. Process for the preparation of a compound comprising a moiety of formula (IX) 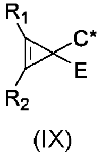
said process comprising the step of reacting a compound of formula (I) as defined in any one of claims 1 to 10 with a compound comprising a moiety of formula C*-M,
wherein C* represents a saturated or unsaturated carbon atom;
M represents a group selected from the group consisting of -MgX being X a halo group, a group of formula -B(OR₁₄)(OR₁₅) wherein R₁₄ and R₁₅ are selected from H and (C₁-C₆)alkyl or; alternatively, R₁₄ and R₁₅ together with the oxygen and boron atoms to which they are attached form a saturated ring optionally substituted at any available position with a (C₁-C₆)alkyl group, and and -ZnR₁₆ wherein R₁₆ is a (C₁-C₆)alkyl group; and wherein said process converts the moiety of formula (C*-M) in the moiety of formula (IX).
